(19) Europäisches Patentamt
European Patent Office
Office européen des brevets

(11) EP 1 392 238 B1

(12) EUROPEAN PATENT SPECIFICATION

(45) Date of publication and mention of the grant of the patent:
**24.08.2005 Bulletin 2005/34**

(21) Application number: **02739645.6**

(22) Date of filing: **03.06.2002**

(51) Int Cl.[7]: **A61K 7/48**

(86) International application number:
**PCT/US2002/017529**

(87) International publication number:
**WO 2002/100374 (19.12.2002 Gazette 2002/51)**

# (54) PERSONAL CARE COMPOSITIONS CONTAINING INVERSE EMULSION POLYMERS

HYGIENEZUSAMMENSETZUNGEN MIT INVERSEN EMULSIONSPOLYMEREN

COMPOSITIONS DE SOINS PERSONNELS CONTENANT DES POLYMERES INVERSEURS D'EMULSION

(84) Designated Contracting States:
**AT BE CH CY DE DK ES FI FR GB GR IE IT LI LU MC NL PT SE TR**

(30) Priority: **08.06.2001 US 297231 P**
**31.05.2002 US 161225**

(43) Date of publication of application:
**03.03.2004 Bulletin 2004/10**

(73) Proprietor: **Noveon IP Holdings Corp.**
**Cleveland, OH 44141-3247 (US)**

(72) Inventors:
- **CHIARELLI, Joseph, A.**
  **Broadview Heights, OH 44147 (US)**
- **SHAH, Pravinchandras, K.**
  **Westlake, OH 44145 (US)**
- **MORAN, Bryan P.**
  **Ohio 44023 (US)**
- **ROUTT, Thomas, A.**
  **Garfield Heights, OH 44125 (US)**
- **AEBISCHER, David, R.**
  **Clinton, OH 44216 (US)**

(74) Representative: **Weber, Thomas et al**
**Patentanwälte**
**von Kreisler-Selting-Werner,**
**Postfach 10 22 41**
**50462 Köln (DE)**

(56) References cited:

| | |
|---|---|
| **EP-A- 1 059 305** | **WO-A-99/15144** |
| **US-A- 4 539 368** | **US-A- 6 136 328** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 392 238 B1

Printed by Jouve, 75001 PARIS (FR)

## Description

### FIELD OF THE INVENTION

This invention relates to use of inverse emulsion polymers having improved electrolyte tolerance and efficiency as emulsifiers, thickeners and stabilizers in personal care compositions. The compositions have excellent shear, shelf and use stability, as well as excellent formulation aesthetics (such as skin feel, residue, moisturizing, emolliency, rub-in, absorption and adsorption characteristics, and the like).

### BACKGROUND OF THE INVENTION

Personal care compositions include after-shave balms, barrier creams, skin whitening compositions, anti-aging creams and lotions, skin moisturizers, cleansers, color cosmetic compositions, foundations, hair conditioners, hair creams and lotions, moisturizers, pomades, sunscreens, toners, and the like. Such compositions can be applied to the skin or hair as creams or lotions (typically as oil-in-water emulsions and sometimes as water-in-oil emulsions), gels (typically containing substantial quantities of water miscible alcohols or glycols), mousses, ointments, pads, pastes, solutions, sprays, sticks (as defined in *Handbook of Cosmetic Science and Technology,* Elsevier Science Publishing, 1993, p.34), and the like. Personal care compositions often use emulsifiers, thickeners, and stabilizers to modify rheological properties and to improve stability. The terms thickener, stabilizer, and emulsifier as used herein are defined in *Handbook of Cosmetic Science and Technology,* Elsevier Science Publishing, 1993, pp. 11 and 116-118; and *Harry's Cosmeticology,* Chemical Publishing Company Inc. 1982, p. 743. The term rheology as used herein is defined in *Harry's Cosmeticology,* Chemical Publishing Company Inc., 1982, pp. 752-753.

Both natural and synthetic materials have been used to modify rheological properties of personal care compositions. Natural materials (such as gum arabic, guar gum, starches and modified starches, and the like) vary widely in quality and thus also in thickening properties. They tend to be prone to microbial attack, which leads to products having limited shelf life. Synthetic materials are used more commonly, since they can be made with more consistent quality and better stability. Synthetic materials used as thickeners in personal care compositions include solid precipitation polymers that generally must be dispersed and have residual acid groups that must be neutralized in order to become effective. Alkali swellable emulsion polymers that are liquid can be more easily mixed in personal care compositions but also have residual acid groups that must be neutralized before such addition.

Inverse emulsion polymers are well known as thickeners in personal care compositions and typically are provided as a water-in-oil emulsion, i.e., a water-dispersible polymer in water that is emulsified in a continuous oil phase. When the inverse emulsion polymer is added to water during preparation of the personal care composition the water phase becomes the continuous phase, and the polymer expands through the water phase to thicken and give stability to the now reversed, oil-in-water, creamy white emulsion.

U.S.-A- 5,216,070 relates to formation of a water-in-oil emulsion of a water-soluble polymer by emulsion polymerization of a 65-85% neutralized water-soluble unsaturated carboxylic acid monomer using a polysiloxane polyalkylene polyether copolymer as an emulsifier. The emulsion remains stable during the course of the polymerization, even though the polymer was not fully neutralized, and can be diluted easily with an aqueous system to invert the emulsion to a neutral oil-in-water emulsion that is useful together with various active ingredients for personal care products.

U.S.-A- 6,051,245 relates to a thickener for personal care products comprising units derived from (a) acrylamide, (b) 2-acrylamido-2-methylpropane-sulfonic acid (AMPS), and (c) a polyfunctional crosslinking monomer present in an amount of from 0.12 to 2 milliequivalents inclusive per mole of total monomer units. Preferred crosslinking monomers are allyl sucrose, allyl pentaerythritol, and $N,N^1$-methylene-bisacrylamide (MBA).

U.S.-A- 6,136,305 relates to a thickener comprising a water-in-oil emulsion and a copolymer having moieties derived from (i) a monounsaturated monocarboxylic acid monomer containing from 3 to 5 carbon atoms, the monocarboxylic acid either being in free form or in the form of an inorganic salt, and (ii) a monoacrylamide monomer. The copolymer is in solution in the aqueous phase constituting the emulsion, and the oil phase consists of a mixture of at least one volatile oil and at least one non-volatile oil in a weight ratio between 90/10 and 10/90.

US-A-6,136,328 relates to a composition in the form of an oil-inwater emulsion with a high electrolyte content, comprising a continuous aqueous liquid phase, a non-aqueous phase and an emulsifying system preferably having an HLB value of greater than or equal to 9. The emulsion may also contain adjuvants, active agents intended in particular to prevent and/or treat skin complaints. The personal care emulsion for topical application described does not contain an electrolyte tolerant inverse emulsion polymer.

WO-A-99/15144 relates to oil-in-water sunscreen compositions and to a method for improving the stability of emulsions employing carboxylic acid polymers, e.g. carbomers and $C_{10}$-$C_{30}$ alkyl acrylates, by neutralizing these polymers with amino methyl propanol and/or DEA-cetyl phosphate. The compositions may further comprise secondary

thickening agents, e.g. polyacrylamide and $C_{13-14}$ isoparaffin and laureth-7, available as Sepigel™, and polymers for aiding the film-forming properties and substantivity of the composition such as copolymer of eicosene and vinyl pyrrolidone. The emulsions described comprise emulsifiers such as Polysorbate 80, surfactants, adjuvants and crosslinkers.

EP-A-1059305 relates to inverse emulsion polymers that are useful in personal care compositions comprising a copolymer or copolymer salt of N,N,-dimethylacrylamide and 2-acrylamido-2-methylpropane sulfonic acid or acid salts in an inert hydrocarbon liquid organic dispersion medium wherein the composition contains an emulsifier/primary surfactant, a chain transfer agent and a cross-linker.

Inverse emulsion polymers of the prior art often have defects. For example, inverse emulsion polymers often contain volatile solvents in the oil phase. Volatile solvents are undesirable because of contribution to air pollution, irritation, and possible toxic effects. Additionally, inverse emulsion polymers tend to have poor electrolyte tolerance. Many adjuvants and formulation aids utilized in personal care compositions are ionic or electrolytic in character. For example, many naturally occurring biologically active and botanical extract adjuvants (e.g., borage extract, black walnut, bee pollen, alfalfa, ginseng, and sea kelp) contain monovalent and/or divalent ions. Poor electrolyte tolerance means that when these ingredients and other electrolyte containing components are added to the inverse emulsion polymer containing personal care composition, the increased ionic or salt content deleteriously affects the thickening ability (i. e., efficiency) of the polymer. Lower efficiency of the polymer system means that more polymer is required to offset the decreased thickening ability of the polymer.

Prior art inverse emulsion polymers also tend to be sensitive to shear. Upon exposure to excessive shear such as homogenization, the prior art emulsions tend to become unstable, i.e., the oil and water phases begin to separate. This lack of stability leads to shorter shelf life and use life of the personal care composition. Special equipment that avoids excessive shear (more than 4,500 rpm) must be used during manufacture of the prior art personal care compositions.

Improved personal care compositions are desired. They must have excellent shear, shelf and use stability, as well as excellent formulation aesthetics (such as skin feel, residue, moisturizing, emolliency, rub-in, and absorption and adsorption characteristics, and the like). Accordingly, it is a general object of this invention to provide personal care compositions that are formulated with inverse emulsion polymers having improved electrolyte tolerance, higher efficiency and function as improved emulsifiers, thickeners and stabilizers.

## SUMMARY OF THE INVENTION

This invention relates to use of inverse emulsion polymers to develop personal care compositions having excellent shear, shelf and use stability, as well as excellent formulation aesthetics (such as skin feel, residue, moisturizing, emolliency, rub-in, and absorption characteristics, and the like). Such personal care compositions are made using inverse emulsion polymers having improved electrolyte tolerance and higher efficiency and that function as improved emulsifiers, thickeners and stabilizers. The personal care compositions comprise (A) topically acceptable liquid phases, and (B) at least one inverse emulsion polymer having a pH from about 5.5 to about 8 and comprising the reaction product of:

(1) at least one complex associative monomer having (a) ethylenic unsaturation in an end group for addition polymerization with monomers (2), (3), and (4), (b) a hydrophilic midsection, and (c) a hydrophobic moiety;

(2) at least one pH sensitive monomer having ethylenic unsaturation and at least one carboxylic group;

(3) an optional copolymerizable non-ionic monomer having ethylenic unsaturation; and

(4) at least one crosslinking monomer.

In particular, the invention is related to a personal care emulsion composition for topical application to the skin comprising:

a) a continuous aqueous liquid phase;
b) a non-aqueous phase;
c) an electrolyte tolerant inverse emulsion polymer composition comprising:

i) a polymer polymerized from a monomer composition comprising (1) at least one pH sensitive monomer having ethylenic unsaturation and containing at least one carboxylic acid moiety, wherein at least 50 wt. % of said monomers containing said carboxylic acid moiety are neutralized; (2) a complex associative monomer represented by the structure:

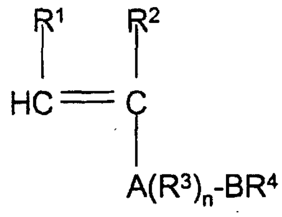

wherein A is selected from a divalent radical represented by -C(O)O-, -CH2C(O)O-, -O-, -CH2O-, NHC(O)O-, -(CH2)m NHC(O)-, -NHC(O)NH-, -C(O)NH-, -C(O)NH(CH2)m-,

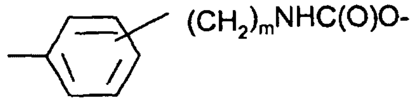

B is selected from a divalent radical represented by -CH2CH2O-, -CH2CH(CH3)O-, -CH2CH2CH2O-, -C(O)-, -(CH2)mNHC(O)-, and -(CH2)mNHC(O)NH-, wherein m represents an integer from 0 to 18; R1 is selected from H, -CH3 and -COOH; R2 is selected from H, -CH3 and -COOH; R3 is selected from -CH2CH2O-, -CH2CH (CH3)O-, - CH2CH2CH2O- and mixtures thereof wherein n is an integer from 1 to about 250; and R4 is selected from substituted and unsubstituted linear and branched C8-C30 alkyl, substituted and unsubstituted C8-C40 alicyclic, C8-C30 alkylaryl wherein the aryl moiety contains 6 to 17 carbon atoms, C6-C17 arylalkyl wherein the alkyl moiety contains 8 to 30 carbon atoms, substituted and unsubstituted C5-C40 carbocylic groups; and (3) a crosslinking monomer; said complex associative monomer is present in the monomer composition in a weight ranging from 0.0001 wt. % to 3 wt. % based on the total weight of monomers present in the composition; and

ii) at least one surfactant having an HLB value less than 7; wherein said polymer maintains at least 50 % of its original Brookfield Viscosity value when 0.1 wt. % of sodium chloride is added to 1 wt. % polymer solids in deionized water (w/w);

d) at least one adjuvant; and

e) a high HLB emulsifier having an HLB value of 7.5 or greater in an amount sufficient to allow said polymer in component to associate with said aqueous phase.

Preferred embodiments will become evident from the attached claims and the detailed description.

## BRIEF DESCRIPTION OF THE DRAWINGS

Fig. 1 is a photomicrograph at 200 magnification showing an oil-in-water emulsion of the polymer composition of the invention wherein the polymer was initially dispersed in the water phase.

Fig. 2 is a photomicrograph at 200 magnification showing an oil-in-water emulsion of the polymer composition of the invention wherein the polymer was initially dispersed in the oil phase.

Fig. 3 is a photomicrograph at 200 magnification showing an oil-in-water emulsion of the polymer composition of the invention wherein the polymer was dispersed in a combined water-in-oil phase and emulsified.

## DETAILED DESCRIPTION OF THE INVENTION

In one aspect of the present invention, the personal care compositions of the present invention comprise (A) a topically acceptable liquid phase, and (B) at least one inverse emulsion polymer having a pH from 5.5 to 8 and comprising the polymerization product of a monomer composition comprising:

(1) 0.0001 wt. % to 3 wt. % (based on total weight of the monomers in the composition) of at least one complex associative monomer having (a) ethylenic unsaturation in an end group for addition polymerization with monomers (2), (3), and (4), (b) a hydrophilic midsection, and (c) a hydrophobic moiety;

(2) 25 wt. % to 99.9998 wt. % (based on total weight of the monomers in the composition) of at least one pH sensitive monomer having ethylenic unsaturation and at least one carboxylic acid group to provide anionic functionality to the polymer;

(3) 0 wt. % to 74.9998 wt. % (based on total weight of the monomers in the composition) of at least one copolymerizable non-ionic monomer having ethylenic unsaturation; and

(4) 0.0001 wt. % to 2 wt. % (based on total weight of the monomers in the composition) of at least one crosslinking monomer.

It is to be recognized that the amount of each of components (1), (2), (3), and (4) is selected from the disclosed range such that the amount of monomer in the polymerizable monomer composition adds up to 100 wt. %.

As used herein, the term "wt. %" means the number of parts by weight of monomer per 100 parts by weight of monomer in the reaction mixture, or the number of parts by weight of ingredient per 100 parts by weight of personal care composition.

## Associative Monomer

The complex associative monomer used in preparing the inverse emulsion polymers typically is a compound including in each molecule: (a) ethylenic unsaturation in an end group for addition polymerization with the other monomers described hereinafter. The complex associative monomer includes a pendant moiety having (b) a hydrophilic portion in the midsection that terminates with (c) a hydrophobic tail portion. The term "complex" means that the monomer is not only hydrophilic or hydrophobic but both hydrophilic and hydrophobic. The term "associative" means that the pendant moiety of the repeating unit polymerized from the complex associative monomer is capable of non-covalent bonding with other portions of the polymer or other ingredients in the personal care composition. Without wishing to be bound by theory of invention it is believed that the non-covalent bonding occurs through hydrogen bonding, Van der Waals forces, etc.

The moiety (a) supplying the vinyl or other ethylenically unsaturated end group for addition polymerization typically is derived from an $\alpha,\beta$-ethylenically unsaturated mono or di-carboxylic acid or the anhydride thereof, preferably a $C_3$ or $C_4$ mono- or di-carboxylic acid or the anhydride thereof. Alternatively, this moiety can be derived from an allyl ether or vinyl ether; a nonionic urethane monomer, such as disclosed in U.S. Reissue Patent No. 33,156 or U.S.-A-5,294,692; or a urea reaction product, such as disclosed in U.S.-A- 5,011,978.

Also included in the chemical structure of each associative monomer molecule is a pendant moiety comprising midsection (b) having a long-chain hydrophilic segment. Typically this midsection comprises a polyoxyalkylene segment of 10 to 250 linear and branched $C_2$ to $C_7$ alkylene oxide repeating units. In another aspect the number can range from 10 to 120 repeating units, and in a further aspect 10 to 60 repeating alkylene oxide repeating units. In still another aspect of the invention, midsections include polyoxyethylene segments, polyoxypropylene segments, and segments comprising mixtures of ethylene oxide and propylene oxide units, all segments comprising 10 to 150 repeating units. In another aspect 10 to 100 repeating units, and in a still further aspect 10 to 60 ethylene oxide units, propylene oxide units, or mixtures of ethylene oxide or propylene oxide units. The mixture of ethylene oxide and propylene oxide units can be random or non-random (e.g., block) sequences.

Examples of associative monomers include those represented by formula I:

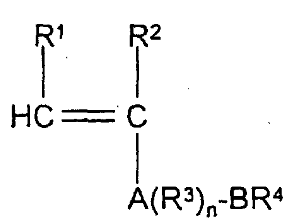

I

wherein A represents -C(O)O-, $-CH_2C(O)O-$, -O-, $-CH_2O-$, -NHC(O)O-, $-(CH_2)_m$ NHC(O)-, -NHC(O)NH-, -C(O) NH-, $-C(O)NH(CH_2)_m-$,

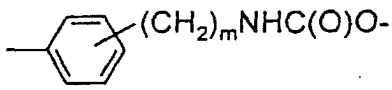

B represents $-CH_2CH_2O-$, $-CH_2CH(CH_3)O-$, $-CH_2CH_2CH_2O-$, -C(O)-, $-(CH_2)_mNHC(O)-$, or $-(CH_2)_mNHC(O)NH-$, wherein m represents an integer from 0 to 18. In another aspect of the invention m represents an integer form 1 to 10, and in a further aspect from 1 to 5.

$R^1$ is H or $CH_3$, or -COOH.

$R^2$ is H or $CH_3$, or -COOH.

$R^3$ is $-CH_2CH_2O-$, $-CH_2CH(CH_3)O-$, $-CH_2CH_2CH_2O-$, or a mixture thereof, wherein n is an integer from 1 to 250. In another aspect of the invention n is an integer from 1 to 100, and in a further aspect from 10 to 80. In a still further aspect n is an integer ranging from about 10 to about 60.

$R^4$ is selected from substituted and unsubstituted linear and branched $C_8$-$C_{30}$ alkyl, substituted and unsubstituted, $C_8$-$C_{30}$ alkylaryl wherein the aryl moiety contains 6 to 17 carbon atoms, substituted and unsubstituted $C_6$-$C_{17}$ arylalkyl wherein the alkyl moiety contains 8 to 30 carbon atoms, and substituted and unsubstituted $C_5$-$C_{40}$ carbocyclic groups. Examples of aryl groups contained in the above described moieties include, but are not limited to, phenyl, naphthyl and anthracenyl. Examples of carbocyclic groups include, but are not limited to, substituted and unsubstituted $C_4$-$C_5$ cycloalkyl, substituted and unsubstituted $C_9$-$C_{40}$ polycycloalkyl arid substituted and unsubstituted $C_9$-$C_{40}$ polycycloalkenyl. When $R^4$ is substituted suitable substituents include, but are not limited to, $C_1$-$C_5$ alkyl, $C_1$-$C_5$ alkoxy, hydroxy, halogen and mixtures thereof. Examples of polycycloalkyl groups include, but are not limited to, groups derived from cholestane and cholestanol. Examples of polycycloalkenyl groups include, but are not limited to, groups derived from sterols; for example, those from animal sources, such as cholesterol, lanosterol, 7-dehydrocholesterol, and the like; vegetation sources, such as phytosterol, stigmasterol, campesterol, and the like; and yeast sources, such as ergosterol, mycosterol, and the like. Since pure cholesterol (such as choleth-24) is expensive, choleth-24 and ceteth-24 (i.e., a blend of cetyl alcohol and cholesterol) or lanolin alcohol (which contains >50% cholesterol) can be used as a less expensive substitute.

Examples of preferred associative monomers include lauryl polyethoxylated methacrylate, palmityl polyethoxylated methacrylate, cetyl polyethoxylated methacrylate, cetylstearyl polyethoxylated methacrylate, stearyl polyethoxylated methacrylate, tristearylphenol polyethoxylated methacrylate, arachidyl polyethoxylated methacrylate, behenyl polyethoxylated methacrylate, cerotyl polyethoxylated methacrylate, montanyl polyethoxylated methacrylate, melissyl polyethoxylated methacrylate, and lacceryl polyethoxylated methacrylate. In one aspect of the invention the foregoing polyethoxylated monomers contain from 10 to 100 ethylene oxide units. In another aspect the polyethoxylated monomers contain from 10 to 60 ethylene oxide units, and in a further aspect contain from 10 to 30 ethylene oxide units.

The amount and ratio of complex associative monomer in the inverse emulsion polymer can vary and depends, among other things, on the final properties desired for the polymer and the process stability. If too much complex associative monomer is used, the polymer becomes too expensive to be practical, and polymerization does not proceed in a stable fashion, producing gels, grits, and the like. For most applications, the amount of complex associative monomer introduced in the reaction mixture for the inverse emulsion polymer should range from 0.0001 wt. % to 3 wt. % based on the total weight of monomer in the reaction mixture. In another aspect the amount of complex associative monomer can range from 0.0001 wt. % to 0.9 wt. % based on the total weight of monomer in the reaction mixture. In another aspect the amount of complex associative monomer can range from 0.001 wt. % to 0.75 wt %, and in a further aspect from 0.001 wt. % to 0.5 wt. % based on the total weight of the monomer in the reaction mixture. In a still further aspect the amount of monomer can range from 0.001 to 0.25 wt. % based on the total weight of the monomer in the polymerizable monomer composition.

**<u>pH Sensitive Monomer</u>**

The inverse emulsion polymer has polymerized therein at least one pH sensitive monomer having ethylenic unsaturation and containing at least one carboxylic acid group. Alternatively, an optional pH sensitive monomer having ethylenic unsaturation and a sulfonic acid group can be copolymerized with the carboxylic acid containing monomer into the inverse emulsion polymer backbone. If utilized, the amount of sulfonic acid containing monomer employed can range from 1 wt. % to 30 wt. % of the total pH sensitive monomer in the polymerizable monomer composition. In another

aspect of the invention, the amount of sulfonic acid monomer employed can range from 5 wt. % to 10 wt. % of the total pH sensitive monomer in the polymerizable monomer composition.

Prior to polymerization, the pH sensitive monomers are neutralized, i.e., the free carboxylic acid groups and the free sulfonic acid groups (if present) are converted to a salt. In one aspect of the invention, 50 to 100 wt. % of the free acid containing monomers are neutralized. In another aspect 60 to 100 wt. % of the free acid containing monomers are neutralized, and in a further aspect 75 to 95 wt. % of the pH sensitive monomers are neutralized. Salts of the pH sensitive monomers can contain any suitable monovalent cation capable of forming a salt with the free carboxylic or free sulfonic acid moiety contained in the monomer. The salts can readily be formed by reacting the free carboxylic acid and free sulfonic acid containing monomers with a cation containing neutralizing base. Suitable bases include but are not limited to, alkali metal (e.g., sodium, potassium, lithium) hydroxides and carbonates, ammonium hydroxide and amine salts.

The term "pH sensitive" means that the charge of the monomer is pH dependent and can change from a neutral molecule to a charged molecule. Suitable carboxylic and sulfonic acid groups include monoacids, diacids, anhydrides of carboxylic acids, half esters of diacids, and the like.

A wide variety of carboxyl-based vinyl monomers can be used. Examples of such monomers include, but are not limited to, acrylic acid, methacrylic acid, itaconic acid, citraconic acid, maleic acid, fumaric acid, crotonic acid, and $C_1$-$C_4$ alkyl half esters of maleic and fumaric, itaconic acid or aconitic acid, such as methyl hydrogen maleate, monoisopropyl maleate and butyl hydrogen fumarate, and the like, and mixtures thereof. In one aspect of the invention, acrylic acid, methacrylic acid, fumaric acid and itaconic acid in mono- or di-acid form, are utilized because of their availability.

Examples of sulfonic acid containing monomers include 2-acrylamido-2-methylpropane sulfonic acid (available as AMPS® from The Lubrizol Corporation), sodium p-styrene sulfonate (available as Spinomar® from The Lubrizol Corporation), sulphoethyl methacrylate, and the like, and mixtures thereof and with the carboxyl-based vinyl monomers.

In one aspect of the invention, the pH sensitive monomer introduced into the monomer reaction mixture. for the inverse emulsion polymer ranges from 25 wt. % to 99.9998 wt. % based on the total weight of the monomer in the monomer reaction mixture. In another aspect, the monomer is present in a range from 60 wt. % to 99.998 wt. %, and in a further aspect from 89.998 wt. % to 99.998 wt. %. based on the total weight of monomer in the reaction mixture.

**Non-Ionic Monomer**

The inverse emulsion polymer has optionally polymerized therein at least one addition copolymerizable non-ionic monomer. Examples of such non-ionic monomers include $C_2$-$C_6$ hydroxy alkyl acrylates and methacrylates, $C_2$-$C_6$ amino alkyl esters of acrylic and methacrylic acids, glycerol monomethacrylate, tris(hydroxymethyl) ethane monoacrylate, pentaerythritol monomethacrylate, N-hydroxymethyl methacrylamide, hydroxyethyl methacrylamide, acrylamide, hydroxypropyl methacrylamide, vinyl caprolactam, N-vinyl pyrrolidone, $C_1$-$C_4$ alkoxy substituted methacrylates and methacrylamides such as methoxyethyl methacrylate, 2(2-ethoxyethoxy)ethyl methacrylate, polyethylene glycol mono methacrylate and polypropylene glycol mono methacrylate, and the like, and mixtures thereof. Acrylamide is preferred.

In one aspect of the invention, the non-ionic monomer introduced in the reaction mixture for the inverse emulsion polymer ranges from 0 wt. % to 74.9998 wt. % of the total weight of monomer in the monomer reaction mixture. In another aspect from 1 wt. % to 39.998 wt. %, and in a further aspect from 1 wt. % to 10 wt. %. based on the total weight of monomer in the reaction mixture.

**Crosslinking Monomer**

The inverse emulsion polymer has polymerized therein at least one polyfunctional crosslinking monomer. By polyfunctional is meant that the crosslinking monomer contains at least two terminal ethylenically unsaturated groups, e.g., $CH_2$=CH-. Examples of suitable crosslinking monomers include any polyene, such as decadiene or trivinyl cyclohexane; acrylamides, such as methylene bis acrylamide; polyfunctional acrylates, such as trimethylol propane triacrylate; polyfunctional vinylidene monomers containing at least 2 terminal $CH_2$=CH- groups, such as butadiene, isoprene, divinyl naphthalene, allyl acrylates and the like; allyl esters, allyl ethers, diallyl esters, dimethallyl ethers, allyl and methallyl acrylates, tetravinyl silane, polyalkenyl methanes, diacrylates, triacrylates, dimethacrylates, trimethacrylates and tetramethacrylates; divinyl compounds such as divinyl benzene, polyallyl phosphate, diallyloxy compounds and phosphite esters, and the like, and mixtures thereof. Examples of specific crosslinking monomers include allyl pentaerythritol, methylene bis acrylamide, allyl sucrose, trimethylolpropane triacrylate, trimethylolpropane trimethacrylate, 1,6-hexanediol diacrylate, trimethylolpropane diallyl ether, pentaerythritol triacrylate, tetramethylene dimethacrylate, ethylene diacrylate, ethylene dimethacrylate, triethylene glycol dimethacrylate, and the like. Preferred crosslinking monomers include methylene bis acrylamide, allyl pentaerythritol, trimethylolpropane diallylether, and allyl sucrose.

In one aspect of the invention, the crosslinking monomer introduced in the reaction mixture for the inverse

emulsion polymer ranges from 0.0001 wt. % to 2 wt. % based on the total weight of monomer in the monomer reaction mixture. In another aspect the amount ranges from 0.001 wt. % to 1 wt. %, and in a further aspect from 0.001 to 0.1 wt. % based on the total weight of monomer in the reaction mixture.

**Preparation of Inverse Emulsion Polymer**

Preparation of water phase

The pH sensitive monomer is dispersed in water and neutralized to the desired level (at least 65 % by wt. of the total amount of pH sensitive monomer in the polymerizable monomer composition) by addition of a suitable neutralizing base, e.g., ammonium hydroxide, sodium hydroxide, potassium hydroxide, sodium carbonate or potassium carbonate. Other ingredients such as chelating agents, chain-transfer agents, comonomers (the complex associative monomer, other pH sensitive monomers, the optional copolymerizable non-ionic monomer, crosslinking monomer, described above) and additional water can be added before, during, or after neutralization. If desired, these ingredients can be added after the water phase is mixed with the oil phase. Alternatively, one or more of these ingredients can be added or metered into the polymerization medium during the polymerization reaction.

Chelating agents that chelate transition metal ions can be added to the water phase. Examples of such compounds include ethylene-diamine tetracetic acid (EDTA) and salts thereof, diethylene triamine pentaacetic acid (DTPA) and salts thereof, ethylene diamine tetramethylene phosphonic acid and salts thereof, citric acid and salts thereof, gluconic acid and salts thereof, Goodrite® 3000X15 chelator available from Noveon, Inc., and mixtures thereof.

It is known that the incorporation of chain transfer agents depresses the molecular weight of the polymer and or alters the molecular structure of the polymer. When desired a small amount of chain transfer agent can be added to the polymerization mixture. Suitable compounds include lower alcohols such as methanol or isopropanol, butylene glycol, phosphate compounds such as sodium hypophosphite, thiol containing monomers, such as 2-mercaptoethanol, 3 mercaptopropionic acid, thioglycolic acid, and formic acid, and the like, and mixtures thereof. The optimum amount required to give chosen polymer properties can be determined by experiment and will depend on, inter alia, the chain transfer agent that is being used. Typically the amount of chain transfer agent employed ranges from 0.001 wt. % to 1% based on total weight of monomer in the reaction mixture.

Preparation of oil phase

Oil is placed in a reactor equipped with a mechanical stirrer, a thermocouple, and a nitrogen inlet. A suitable amount of low HLB (3 to 6.9) surfactant or mixture of low HLB surfactants can be mixed into the oil. Suitable low HLB surfactants include, but are not limited to, ethoxylated saturated $C_{12}$-$C_{22}$ acid alcohols, ethoxylated unsaturated $C_{12}$-$C_{22}$ acid alcohols, $C_8$-$C_{22}$ alcohol ethoxylates, ethoxylated vegetable derived oils, ethoxylated vegetable derived esters, vegetable derived esters, ethoxylated $C_8$-$C_{22}$ fatty acids, silicone glycol copolymers, esters of sorbitol, ethoxylated esters of sorbitol, block copolymers of ethyleneoxide/propyleneoxide, and alkyl phenol ethoxylates. Other suitable low HLB surfactants for inverse emulsion polymerization are well known to those skilled in the art and include surfactants such as those listed in U.S.-A- 4,650,827 (see column 4, lines 46-60), as well as those low HLB surfactants listed on pages 249, 250, 251, and 252 of *MeCutcheon's Detergents and Emulsifiers,* North American Edition, Allured Publishing Corporation, 1996. The meaning of the term "HLB" is well understood by those skilled in the art and is defined in *Surfactants and Interfacial Phenomena,* $2^{nd}$ Edition, pp. 326-329, John Wiley and Sons, 1989. The surfactant serves to facilitate reduction of the particle size of the aqueous monomer droplets and to stabilize the water-in-oil emulsion. The amount of low HLB surfactant employed can range from 0.1 wt. % to 10 wt. % of the weight of the total monomer composition in the reaction medium. In another aspect of the invention, the amount of low HLB surfactant can range from 0.5 wt. % to 5 wt. %, and in a further aspect from 1 wt. % to 3 wt. % of the weight of the total amount of monomer in the polymerizable monomer composition.

Suitable oils for use in the oil phase are various types of water inert insoluble organic liquids. Personal care grade mineral oils, such as Carnation® white mineral oils, Petro-Canada Oils, Witco Chemical light mineral oils, Drakeol® mineral oils from Penreco Inc., polyisobutene, isohexadecane, caprylic/capric triglycerides, cetearyl octanoate, $C_{12}$-$C_{14}$ alkyl benzoate, vegetable oils, and the like, having specific gravity in the range of 0.78 to 0.95 at 25°C and a distillation point at of 160°C or higher.

Preparation of water-in-oil emulsion

While the oil phase is being agitated, the water phase is added to the oil phase. Depending on the particle size and size distribution desired, a high shear mixing device such as a homogenizer or a blender can be used to make the water-in-oil emulsion.

Polymerization

The polymerization reaction is carried out under inert atmosphere conditions with minimal exposure to oxygen. Polymerization is conducted using a free radical initiator to start the polymerization. Both water-soluble free radical initiators, such as persulfates, and oil-soluble free radical initiators, such as azo and peroxide thermal initiators, can be used. If a low temperature initiation is desired, then a redox free radical initiation system also can be used. A suitable amount of initiator, typically 0.001 wt. % to 1 wt. % based on the total weight of monomers in the reaction mixture is added. The polymerization is continued until no appreciable heat is released from the polymerization. Additional initiator (s) can be introduced into the reaction vessel in order to further drive the monomer conversion to completion. The conversion of monomer to polymer is essentially quantitative. In other words, each mole of monomer is essentially completely converted to polymer. Monomer conversion is 99 % or greater.

Surprisingly and unexpectedly, the foregoing inverse emulsion polymer composition demonstrates an improvement in electrolyte or salt tolerance when used in topical personal care compositions containing monovalent and or divalent electrolytes. Many adjuvants and formulation aids utilized in personal care compositions contain mono- and divalent electrolytes. Electrolytes are deleterious to the emulsifying, stabilizing and thickening properties of inverse emulsion polymers contained in personal care compositions. Electrolyte or salt tolerance is defined herein as the ability of the polymer to maintain a substantial viscosity and emulsion stability when a substantial amount of monovalent or divalent ions are added to the composition (See Tables 17, 18, 19, and 20 in the examples).

In one aspect the inverse emulsion polymer(s) of the present invention maintains at least 50 % of its original or initial viscosity when 0.1 wt. % of NaCl is added to 1 wt. % of polymer in deionized water (w/w). In further aspect the polymer(s) maintains 60 % of its original viscosity, and in a still further aspect the polymer(s) maintains 70 % of its original viscosity when about 0.1 wt. % of NaCl is added to 1 wt. % of polymer in deionized water (w/w basis).

In another aspect of the invention the instant inverse emulsion polymer(s) maintains at least 25% of its original or initial viscosity when 0.25 wt. % of NaCl is added to 1 wt. % of polymer in deionized water (w/w basis). In another aspect the polymer(s) maintains 40% of its original viscosity, and in a still further aspect the polymer(s) maintains 50% of its original viscosity when 0.25 wt. % of NaCl is added to 1 wt. % of polymer in deionized water (w/w basis). Methods for determining the salt tolerance of the polymers of the present invention are discussed below.

## Topically Acceptable Composition

Liquid Phases

One aspect of the present invention concerns an electrolyte tolerant personal care composition that has improved shear, shelf and use stability, as well as excellent formulation aesthetics (such as skin feel, residue, moisturizing, emolliency, rub-in, and absorption characteristics, and the like). The topically acceptable composition of the present invention is described below. The term "topically acceptable composition" means a composition comprising an aqueous phase, non-aqueous phase, high HLB surfactant(s), inverse emulsion polymer composition and personal care adjuvants and/or a formulation aid(s) that may be applied directly to the body. The personal care adjuvant(s) can be carried in the aqueous phase, in the non-aqueous phase or in both the aqueous and the non-aqueous phases.

The oil phase and aqueous phase utilized to prepare the inverse emulsion polymer(s) of the invention serve as the base phases for the non-aqueous and aqueous phases of the personal care compositions of the invention. Additional non-aqueous (e.g., oils, non-aqueous solvent, etc.) and aqueous phase (e.g., water, distilled water, deionized water, etc,) components are optionally incorporated into the personal care compositions as diluents and/or carriers for the personal care adjuvant(s) and formulation aid(s) described below.

The non-aqueous phase can include any composition of water immiscible substances known to those skilled in the art. Non-aqueous phase components include but are not limited to oils and solvents derived from synthetic or natural origin, such as oils derived from plants and vegetables (e.g. sunflower seed oil, hydrogenated castor seed oil), silicone oils, fluorinated hydrocarbon oils, hydrocarbon oils, mineral oils (white mineral oils, Petro-Canada Oils, light mineral oils, Drakeol® mineral oils from Penreco Inc), polyisobutene, hydrogenated polyisobutylene, isohexadecane, caprylic/capric triglycerides, cetearyl octanoate, $C_{12}$-$C_{15}$ alkyl benzoate, and mixtures thereof. This phase can contain a water immiscible personal care adjuvant(s) and/or formulation aid(s) described below that can be suspended, dispersed, solubilized, or have the property of miscibility in the non-aqueous phase. In one aspect of the invention, the non-aqueous phase typically comprises 1 wt. % to 90 wt. % of the personal care composition. In another aspect the non-aqueous phase is present from 1 wt. % to 80 wt. %, and in a further aspect 5 wt. % to 70 wt. % of the personal care composition.

The aqueous phase includes water and can contain a personal care adjuvant(s) and/or formulation aid(s) described below that are soluble, suspendable, dispersible, swellable, or miscible in the aqueous phase. In one aspect of the invention, the aqueous phase comprises from 10 wt. % to about 99 wt. % of the personal care composition. In

another aspect from 20 wt. % to 99 wt. % and in a further aspect from 30 wt. % to 95 wt. % of the personal care composition.

A personal care adjuvant comprises a substance that imparts a desired personal care property to the topical composition. Personal care adjuvants include but are not limited to humectants, emollients, fragrances, biologically active materials, botanical extracts, conditioners, sunscreens, pharmaceutical actives, conditioning polymers, vitamins, cleansing surfactants, and the like.

In addition to personal care adjuvants, one or more optional formulation aids can be included in the topically acceptable composition. These ingredients include, but are not limited to, preservatives, opacifiers, rheology modifiers, emulsifiers, chelating agents, neutralizing agents and pH adjusters, spreading aids, viscosity adjusters, and colorants as well as numerous other optional components for enhancing and maintaining the properties of the personal care compositions.

High HLB Surfactant

High HLB surfactant(s) can be added in order to aid the water in oil emulsion to accept water and release the polymer to the formulation continuous phase (i.e. water). High HLB surfactants are those having a HLB number of 7.5 or greater as defined in *McCutcheon's Detergents and Emulsifiers*, North American Edition, Allured Publishing Corporation, 1996. Suitable high HLB surfactants include, but are not limited to, $C_8$-$C_{22}$ alcohol ethoxylates, ethoxylated vegetable derived oils, ethoxylated $C_8$-$C_{22}$ fatty acids, $C_8$-$C_9$ alkyl phenol ethoxylates, esters of sorbitol, ethoxylated esters of sorbitol, and mixtures thereof. Additional examples of high HLB surfactants are disclosed in pages 253-263 of *McCutcheon's Detergents and Emulsifiers, supra.* The high HLB surfactant(s) is utilized in an amount sufficient to facilitate the association (i.e., contact) of the inverse emulsion polymer with the aqueous phase of the personal care composition. The high HLB surfactant can be employed in a range from 0.1 wt. % to 10 wt. % of the total wt. of the monomers utilized in the polymerizable monomer composition. In another aspect of the invention from 0.5 to 5 wt. %, and in a further aspect from 1 wt. % to 3 wt. % of high HLB surfactant can be employed.

Exemplary skin care compositions utilizing personal care adjuvants and formulation aids are disclosed in U. S.-A- 5,073,372; 5,380,528; 5,599,549; 5,874,095; 5,883,085; 6,013,271; and 5,948,416.

Such components are also described in detail in well known references such as Mitchell C. Schlossman, *The Chemistry and Manufacture of Cosmetics*, Volumes I and II, Allured Publishing Corporation, 2000.

To those skilled in the art it is known that emulsifiers may serve multiple functions typical of the personal care adjuvants described above. Emulsifiers can function as emollients and delivery vehicles for components of the personal care compositions in addition to the intended purpose of altering the surface tension of water. Emulsifiers have a balance of hydrophilic and lipophilic properties such that they are often found at the interface of the non-aqueous phase and the aqueous phase, sharing the properties of both phases.

The above described non-aqueous phase, aqueous phase, high HLB surfactant(s), one or more personal care adjuvants and one or more optional formulation aids are combined with the salt tolerant inverse emulsion polymer composition to form the desired personal care composition. The amount of inverse emulsion polymer (i.e., neat polymer) in the topically acceptable personal care composition ranges from 0.01 wt. % to 10 wt. % of the total weight of the personal care composition. In another aspect the amount of polymer can range from 0.01 wt. % to 8 wt. %, and in a further aspect from 0.1 wt. % to 5 wt. % of the total weight of personal care composition of the present invention. The balance of the personal care composition comprises a non-aqueous phase, an aqueous phase, emulsifier, personal care adjuvants and optional formulation aids.

Personal care compositions are well known to those skilled in the art and include after-shave balms, barrier creams, skin whitening compositions, anti-aging emulsions, skin moisturizers, cleansers, color cosmetic compositions, foundations, hair conditioners, hair creams and lotions, moisturizers, pomades, sunscreens, toners, and the like. Such compositions can be applied to the skin or hair as creams, gels, mousses, ointments, pads, pastes, solutions, sprays, sticks, and the like. These compositions typically are formulated as creams or lotions (oil-in-water emulsions and sometimes water-in-oil emulsions), gel creams, or as gels that can contain substantial quantities of water miscible alcohols or glycols. The inverse emulsion polymers described heretofore function as emulsifiers, thickeners and stabilizers to modify rheological properties and to improve stability of the personal care compositions.

**Personal Care Adjuvants**

Humectants

Humectants are defined as materials that absorb or release water vapor, depending on the relative humidity of the environment, *(Harry's Cosmeticology*, Chemical Publishing Company Inc., 1982 p. 266). Suitable humectants that can be included in the topically acceptable personal care composition include, but are not limited to, allantoin;

pyrrolidonecarboxylic acid and its salts; hyaluronic acid and salts thereof; sorbic acid and salts thereof; urea, lysine, arginine, cystine, guanidine, and other amino acids; polyhydroxy alcohols such as glycerin, propylene glycol, hexylene glycol, hexanetriol, ethoxydiglycol, dimethicone copolyol, and sorbitol, and the esters thereof; polyethylene glycol; glycolic acid and glycolate salts (e.g. ammonium and quaternary alkyl ammonium); chitosan; aloe-vera extracts; algae extract; honey and derivatives thereof; inositol; lactic acid and lactate salts (e.g. ammonium and quaternary alkyl ammonium); sugars and starches; sugar and starch derivatives (e.g. alkoxylated glucose); D-panthenol; magnesium ascorbyl phosphate, arbutin, kojic acid, lactamide monoethanolamine; acetamide monoethanolamine; and the like, and mixtures thereof. Humectants also include the $C_3$-$C_6$ diols and triols, such as glycerin, propylene glycol, hexylene glycol, hexanetriol, and the like, and mixtures thereof. When utilized, humectants typically comprise 1 wt. % to 10 wt. % of the total weight of the personal care compositions of the present invention. In another aspect the amount can range from 2 wt. % to 8 wt. %, and in a further aspect from 3 wt. % to 5 wt. % of the total weight of the personal care composition.

Emollients

An emollient is defined as a substance which regulates the rate and quantity of water uptake by the skin (*Handbook of Cosmetic Science and Technology,* Elsevier Science Publishing, 1993, p. 175). Emollients that can be included in the topically acceptable personal care composition include, but are not limited to; mineral oil; stearic acid; fatty alcohols such as cetyl alcohol; cetearyl alcohol; myristyl alcohol; behenyl alcohol; and lauryl alcohol; cetyl acetate in acetylated lanolin alcohol; isostearyl isostearate; geurbet esters; geurbet alcohols; octyl stearate; isostearyl benzoate; dicaprylyl maleate; caprylic and capric triglyceride; petrolatum; lanolin and derivatives thereof; coco butter; shea butter; ethoxylated beeswax; beeswax and esters there of; silicone ester ethoxylates; fatty alcohol ether ethoxylates such as ceteareth-20; oleth-5; and ceteth-5; avocado oil or glycerides; sesame oil or glycerides; safflower oil or glycerides; sunflower oil or glycerides; botanical seed oils; palm kernel oil and glycerides; almond oil and glycerides; volatile silicone oils; non-volatile emollients, and the like; and mixtures thereof. Suitable non-volatile emollients include fatty acid and fatty alcohol esters, highly branched hydrocarbons, and the like, and mixtures thereof. The fatty acid and fatty alcohol esters include decyl oleate, butyl stearate, myristyl myristate, octyldodecyl stearoylstearate, octylhydroxystearate, di-isopropyl adipate, isopropyl myristate, isopropyl palmitate, ethyl hexyl palmitate, isodecyl neopentanoate $C_{12}$-$C_{15}$ alcohol benzoate, diethyl hexyl maleate, PPG-14 butyl ether and PPG-2 myristyl ether propionate, cetearyl octanoate, and the like, and mixtures thereof. Suitable highly branched hydrocarbons include isohexadecane, and the like, hydrogenated polyisobutene, polyisobutene, and mixtures thereof. Also included are volatile silicones, such as cyclic or linear polydimethylsiloxanes, and the like. The number of silicon atoms in cyclic silicones can range from 3 to 7 in one aspect of the invention, and in another aspect from 4 to 5. Exemplary volatile silicones, both cyclic and linear, are available from Dow Coming Corporation as Dow Coming 344, 345, and 200 fluids; Union Carbide as Silicone 7202 and Silicone 7158; and Stauffer Chemical as SWS-03314.

The linear volatile silicones typically have viscosities of less than 5 cP at 25°C., while the cyclic volatile silicones typically have viscosities of less than 10 cP at 25°C. "Volatile" means that the silicone has a measurable vapor pressure. A description of volatile silicones can be found in Todd and Byers, "Volatile Silicone Fluids for Cosmetics", *Cosmetics and Toiletries,* Vol. 91, January 1976, pp. 27-32. Other suitable *emollients* include polydimethylsiloxane gums, aminosilicones, phenylsilicones, polydimethyl siloxane, polydiethylsiloxane, polymethylphenylsiloxane, polydimethylsiloxane gums, polyphenyl methyl siloxane gums, amodimethicone, trimethylsilylamodimethicone, diphenyldimethyl polysiloxane gums, and the like. In one aspect of the invention, if present, the emollients employed (alone or in combination), range from 1 wt. % to 20 wt. %, of the total weight of the personal care composition of the present invention..In another aspect the weight of the emollient can range from 2 wt. % to 15 wt. %, and in a further aspect from 3 wt. % to 10 wt. % of the total weight of the personal care composition.

Pharmaceutical Actives

Pharmaceutical actives that can be included in the topically acceptable personal care composition can be selected from any chemical substance, material or compound suitable for topical administration and induces a desired pharmacological local or systemic effect. Such actives include, but are not limited to, antibiotics, antiviral agents, analgesics (e.g. ibuprofen, acetyl salicylic acid, naproxen, and the like), antihistamines, anti-inflammatory agents, antipruritics, antipyretics, anesthetic agents, diagnostic agents, hormones, antifungals, antimicrobials, cutaneous growth enhancers, pigment modulators, antiproliferatives, antipsoriatics, retinoids, anti-acne medicaments (e.g., benzoyl peroxide, sulfur, and the like), antineoplastics agents, phototherapeutic agents, and keratolytics (e.g. resorcinol, salicylic acid, and the like), and the like, and mixtures thereof. When utilized the pharmaceutical active typically comprises from 0.1 wt. % to 20 wt. % of the total weight of the personal care compositions of the present invention.

Botanical Extracts

Botanical extracts are defined as extracts from botanicals that can be obtained via various preparations including tincture, fluid extract, solid extract, powdered extract, homeopathic dilution, oil extract, native extract, aqueous extract, and the like. Properties of these preparations are described in *Botanicals A Phytocosmetic Desk Reference,* Frank S. D'Amelio, Sr., CRC Press LLC, 1999, p. 39). Botanical extracts include, but are not limited to, aloe vera, adders toungue, alder, alfalfa, apple, artichoke, avena, barberry, bearberry, bee pollen, bilberry, black walnut, borage, calendula, capsicum, chamomile, clove, cucumber, coriander fruit, gensing, ginger, ginko, gotukola, green tea, henna, honey, horse chestnut, jasmine flowers, kelp, lemon grass, licorice root, marigold, oats, orange blossom, papaya, paper mulberry, periwinkle, plaintain, rose, rose hips, rosemary, sage, sandalwood, seaweed, spirulina, tea tree oil, walnut, wheat grass, witch hazel, yohimbe, and the like. Botanical extracts when utilized in the present personal care compositions comprise from 0.05 wt. % to 2 wt. % of the total weight of the personal care composition.

Sunscreens

Suitable sunscreens that can be included in the topically acceptable personal care composition must be used in safe and photoprotectively effective amounts in the personal care compositions of the present invention. Exemplary sunscreens include those set forth in Segarin et al., *Cosmetics Science and Technology*, at Chapter VIII, pages 1890 et. seq., as well as 64 Fed. Reg. 27666-27693 (May 21, 1999). Specific sunscreening agents include, for example, p-aminobenzoic acid and its salts and derivatives (ethyl, isobutyl, glyceryl esters; p-dimethylaminobenzoic acid; 2-ethylhexyl-N,N-dimethylaminobenzoate); anthranilates (i.e., o-aminobenzoates; methyl, octyl, amyl, menthyl, phenyl, benzyl, phenylethyl, linalyl, terpinyl, and cycohexenyl esters); salicylates (octyl, amyl, phenyl, benzyl, menthyl, glyceryl, and dipropyleneglycol esters); cinnamic acid derivatives (ethylhexyl-p-methoxy; menthyl and benzyl esters, -phenyl cinnamonitrile; butyl cinnamoyl pyruvate); dihydroxycinnamic acid derivatives (umbelliferone, methylumbelliferone, methylaceto-umbelliferone); trihydroxycinnamic acid derivatives (esculetin, methylesculetin, daphnetin, and the glucosides, esculin and daphnin); hydrocarbons (diphenylbutadiene, stilbene); dibenzalacetone and benzalacetophenone; naphtholsulfonates (sodium salts of 2-naphthol-3,6-disulfonic and of 2-naphthol-6,8-disulfonic acids); dihydroxynaphthoic acid and its salts; o- and p-hydroxybiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives (8-hydroxyquinoline salts, 2-phenyl quinoline); hydroxymethoxy-substituted benzophenones; uric and vilouric acids; tannic acid and its derivatives (e.g. hexaethylether); (butyl carbityl) (6-propyl piperonyl) ether; hydroquinone; benzophenones (oxybenzone, sulisobenzone, dioxybenzone, benzoresorcinol, 2,2',4,4'-tetrahydroxybenzophenone, 2,2'-dihydroxy-4,4'-dimethoxybenzophenone, octabenzone; 4-isopropyldibenzoylmethane; butylmethoxyldibenzoylmethane; octocrylene; 4-isopropyl-dibenzoylmethane; and camphor derivatives such as methylbenzylidene or benzylidene camphor; and the like, and mixtures thereof. Other sunscreens include the inorganic sunblocks such as titanium dioxide (micronized titanium dioxide, 0.03 μm), zinc oxide, silica, iron oxide and dioxide, and the like, and mixtures thereof with one another and with the aforementioned organic sunscreens. Without being limited by theory, it is believed that these inorganic materials provide a sunscreening benefit through reflecting, scattering, and absorbing harmful UV, visible, and infrared radiation. Particularly useful are the sunscreens ethylhexyl-p-methoxycinnamate and benzophenone, either alone, as a mixture, or in combination with the physical sunscreen titanium dioxide.

By "safe and photoprotectively" is meant an amount of sunscreen sufficient to provide photoprotection when the composition is applied, but not so much as to cause any side effects such as skin reactions. When employed in the compositions of the present invention the sunscreens comprise from 0.5 wt. % to 50 wt. %, of the total weight of the skin care compositions. In another aspect from 0.5 wt. % to 30 wt. %, and in a further aspect from 0.5 wt. % to 20 wt. % of the total weight of the skin care compositions of the present invention. Exact amounts will vary depending upon the sunscreen chosen and the desired amount of Sun Protection Factor (SPF).

SPF is a commonly used measure of photoprotection of a sunscreen against erythema. This number is derived from another parameter, the minimal erythemal dose (MED). MED is defined as the least exposure dose at a specified wavelength that will elicit a delayed erythema response. The MED indicates the amount of energy reaching the skin and the responsiveness of the skin to the radiation. The SPF of a particular photoprotector is obtained by dividing the MED of protected skin by the MED of unprotected skin. The higher the SPF, the more effective the agent in preventing sunburn. The SPF value tells how many times longer a person can stay in the sun with use of the sunscreen (compared to the same person with unprotected skin) before that person will experience 1 MED. For example, utilizing a sunscreen with an SPF of 6 will allow an individual to stay in the sun six times longer before receiving MED. As the SPF value of a sunscreen increases, a lesser chance exists for development of tanning of the skin.

Cleansing Surfactants

Cleansing surfactants that can be included in the topically acceptable personal care composition include a wide variety of nonionic, cationic, anionic, and zwitterionic surfactants, such as those disclosed in *McCutcheon's Detergents and Emulsifiers*, North American Edition (1996), Allured Publishing Corporation, and in U.S.-A- 3,755,560; 4,421,769; 4,704,272; 4,741,855; 4,788,006; and 5,011,681. Examples of suitable surfactants include, but are not limited to, alkyl and alkenyl sulfates; alkyl and alkenyl ethoxylated sulfates (in one aspect of the invention having an average degree of ethoxylation ranging from 1 to 10); succinamate surfactants such as alkylsulfosuccinamates and dialkyl esters of sulfosuccinic acid; neutralized fatty acid esters of isethionic acid; and alkyl and alkenyl sulfonates, such as olefin sulfonates and beta-alkoxy alkane sulfonates; and the like. In one aspect of the invention alkyl and alkenyl sulfates and alkyl and alkenyl ethoxylated sulfates, e.g., the sodium and ammonium salts of $C_{12}$-$C_{18}$ sulfates and ethoxylated sulfates (in one aspect of the invention having a degree of ethoxylation ranging from 1 to 6, and in a further aspect from 1 to 4), such as lauryl sulfate and laureth (3.0) sulfate; sodium 3-dodecylaminopropionate; N-alkyltaurines such as prepared by reacting dodecylamine with sodium isethionate according to the teaching of U.S.-A-2,658,072; N-higher alkyl aspartic acids such as produced according to the teaching of U.S.-A- 2,438,091; and products sold under the trade name "Miranol" described in U.S.-A- 2,528,378. and the like. Other suitable surfactants include $C_6$-$C_{22}$ alkyl amphoglycinates and $C_6$-$C_{22}$ alkyl amphopropionates in one aspect and in another aspect $C_8$-$C_{12}$ alkyl amphoglycinates and amphopropionates; and the like. Mixtures of the foregoing surfactants can also be used.

Suitable zwitterionic cleansing surfactants for use in the present personal care compositions include, but are not limited to, linear and branched $C_8$-$C_{18}$ aliphatic quaternary ammonium, phosphonium, and sulfonium compounds that include a substituent having an anionic water-solubilizing group, such as carboxy, sulfonate, sulfate, phosphate, phosphonate, and the like. Classes of zwitterionics include alkyl amino sulfonates, alkyl betaines and alkyl amido betaines, stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles ethylene oxide) stearylamine, dihydroxy ethyl stearylamine, arachidylbehenylamine, and the like. Mixtures of the foregoing can also be used. In one aspect of the invention the cleansing surfactants, comprise from 0 wt. % to 25 wt. %, of the total weight of the personal care composition. In another aspect the amount ranges from 0.5 wt. % to 20 wt. %, and in a further aspect from 1 wt. % to 12 wt. % of the total weight of the personal care compositions of the present invention.

Skin Conditioning Polymers

Skin conditioning polymers that can be included in the topically acceptable personal care composition include, but are not limited to, quaternized guar gum, quaternized cellulosics, polyquatemium 4, polyquaternium 7, polyquaternium 10, polyquatemium 11, polyquaternium 39, polyquaternium 44, and the like, and mixtures thereof. In one aspect of the invention, the conditioning agents, if present, comprise 0.01 wt. % to 3 wt. % of the total weight of the personal care composition. In another aspect the amount can range from 0.1 wt. % to 2 wt. %, and in a further aspect from 0.1 wt. % to 1 wt. % of the total weight of the skin care compositions of the present invention.

Vitamins

Various vitamins that can be utilized in the topically acceptable personal care composition include, but are not limited to vitamin A, vitamin B, biotin, pantothenic acid, vitamin C, vitamin D, vitamin E, tocopherol acetate, retinyl palmitate, magnesium ascorbyl phosphate, and the like, and derivatives, and mixtures thereof. In one aspect of the invention the amount of vitamin(s) that can be employed in the personal care compositions of the invention range from 0.001 wt. % to 5 wt. % of the total personal care composition. In another aspect the amount can range from 0.01 wt. % to 2 wt. %, and in a further aspect 0.1 wt. % to 1 wt. % of the total personal care composition.

**Formulation Aids**

Chelating agents

Chelating agents that can be utilized in the topically acceptable personal care composition include, but are not limited to, EDTA (ethylenediamine tetraacetic acid) and salts thereof such as disodium EDTA, citric acid and salts thereof, cyclodextrins, and the like, and mixtures thereof. In one aspect of the invention, chelating agents comprise from 0.001 wt. % to 3 wt. % of the personal care composition of the invention. In another aspect the amount can range from 0.01 wt. % to 2 wt. %, and in a further aspect from 0.01 wt. % to 1 wt. % of the total weight of the personal care composition.

Diluent

Additional diluents can be included in the topically acceptable personal care composition to adjust the amount of the aqueous and/or non-aqueous liquid phases in the personal care composition. Water and/or the non-aqueous phase media discussed previously can be added to the personal care composition to bring these phases within the disclosed ranges.

Neutralizers and pH Adjusters

Neutralizers and pH adjusters can be included in the topically acceptable personal care composition to bring the pH of the personal care composition to desired levels. Suitable neutralizers and pH adjusters include, but are not limited to, triethanolamine, aminomethyl propanol, ammonium hydroxide, sodium hydroxide, other alkali hydroxides, borates, phosphates, pyrophosphates, cocamine, oleamine, diisopropanolamine, diisopropylamine, dodecylamine, PBG-15 cocamine, morpholine, tetrakis(hydroxypropyl)ethylenediamine, triamylamine, triethanolamine, triethylamine, tromethamine (2-Amino-2-Hydroxymethyl-1,3-propanediol, ascorbic acid and salts thereof, sorbic acid and salts thereof, phosphoric acid and salts thereof, citric acid and salts thereof, lactic acid and salts thereof, glycolic acid and salts thereof, boric acid and salts thereof, acetic acid and salts thereof, and the like, and mixtures thereof. In one aspect of the invention neutralizers or pH adjusters are utilized in the personal care composition of the invention in an amount sufficient to impart a pH ranging from 4 to 10. In another aspect the pH adjusters are utilized in an amount sufficient to impart a pH ranging from 4.5 to 8, and in a further aspect from 5 to 7.5.

Opacifiers

If desired, opacifiers can be included in the topically acceptable personal care composition. These include, but are not limited to, glycol fatty acid esters such as glycol dibehenate, glycol dioleate, glycol distearate, glycol ditallowate, glycol hydroxystearate, glycol montanate, glycol palmitate, and glycol stearate; fatty acids and hydrogenated fatty acid blends such as behenic acid, arachidic acid, palmitic acid, myristic acid, corn acid, palm acid, palm kernel acid, hydrogenated coconut acid, hydrogenated menhaden acid, hydrogenated palm acid, hydrogenated tallow acid, alkoxylated fatty acid esters; silica; alkanolamides such as behenamide, linoleamide, and stearamide; talc; nylon, fatty acid alcohols such as arachidyl alcohol, behenyl alcohol, stearyl alcohol, cetyl alcohol, and myristyl alcohol; waxes and oils; kaolin; magnesium silicate; titanium dioxide; and the like, and mixtures thereof. Opacifiers, if utilized, comprise from 0.1 wt. % to 8 wt. % of the total weight of the personal care composition. In another aspect the amount ranges from 0.5 wt. % to 6 wt. %, and in a further aspect from 1 wt. % to 5 wt. % of the total weight of the personal care compositions of the present invention.

Preservatives

The preservatives that can be included in the topically acceptable personal care composition are, but are not limited to, polymethoxy bicyclic oxazolidine, methylparaben, propylparaben, ethylparaben, butylparaben, benzoic acid and the salts of benzoic acid, benzyltriazole, DMDM hydantoin (also known as 1,3-dimethyl-5,5-dimethyl hydantoin), imidazolidinyl urea, phenoxyethanol, phenoxyethylparaben, inethylisothiazolinone, methylchloroisothiazolinone, benzoisothiazolinone, triclosan, sorbic acid, salicylic acid salts, and the like, and mixtures thereof. In one aspect of the invention the preservative(s), if present, comprises 0.01 wt. % to 1.5 wt. % of the total weight of the personal care composition. In another aspect the amount of preservative ranges from 0.1 wt. % to 1 wt. %, and in a further aspect from 0.3 wt. % to 1 wt. % of the total weight of the personal care compositions of the present invention.

Spreading Aids

The spreading aids that can be included in the topically acceptable personal care composition are, but are not limited to, hydroxypropyl methylcellulose, hydrophobically modified cellulosics, xanthan gum, cassia gum, guar gum, locust bean gum, dimethicone copolyols of various degrees of alkoxylation, boron nitride, talc, and the like, and mixtures thereof. In one aspect of the invention the spreading aid(s), if present, comprises 0.01 wt. % to about 5 wt. % of the total weight of the personal care composition. In another aspect the amount ranges from 0.1 wt. % to 3 wt. %, and in a further aspect from 0.1 wt. % to 2.0 wt. % of the total weight of the personal care compositions of the present invention.

Rheology Modifiers

The rheology modifiers that_can be included in the topically acceptable personal care composition include synthetic polymers and natural thickeners. Exemplary synthetic polymers include, but are not limited to, carbomers, acrylates/$C_{10}$-$C_{30}$ alkyl acrylate crosspolymer, acrylates copolymer, polyacrylamide and $C_{13}$-$C_{14}$ isoparaffin and laureth-7 (supplied as SEPIGEL® 305 by SEPPIC), acrylamides copolymer and mineral oil and $C_{13}$-$C_{14}$ isoparaffin and polysorbate 85 (supplied as SEPIGEL® 501 by SEPPIC), $C_{13}$-$C_{14}$ isoparaffin and isostearyl isostearate and sodium polyacrylate and polyacrylamide and polysorbate 60 (supplied as SEPIGEL® 502 by SEPPIC), acrylamide/sodium acryloyldimethyltaurate copolymer and isohexadecane and polysorbate 60 (supplied as Simulgel® 600 by SEPPIC), sodium polyacryloyldimethyltaurate and isohexadecane and sorbitan oleate (supplied as Simulgel® 800 by SEPPIC), ammonium polyacrylate and isohexadecane and PEG-40 castor oil (supplied as Simulgel® A by SEPPIC), sodium acrylate/acryloyldimethyltaurate copolymer and isohexadecane and polysorbate 80 (supplied as Simulgel® EG by SEPPIC), sodium acrylate/acryloyldimethyltaurate copolymer and polyisobutene and caprylyl/capryl glucoside (supplied as Simulgel® EG-SL by SEPPIC), hydroxyethyl acrylate/sodium acryloyldimethyltaurate copolymer and squalane and polysorbate 60 (supplied as Simulgel® NS by SEPPIC), hydrophobically modified alkali swellable emulsion polymers (HASE).

Natural thickeners include, but are not limited to, cellulosics and hydrophobically modified cellulosics, xanthan gum, guar gum, cassia gum, and aluminum magnesium silicate. In one aspect of the invention the rheology modifiers, if utilized, comprise from 0.01 wt. % to 5 wt. % of the total weight of the personal care composition. In another aspect the amount ranges from 0.1 wt. % to 3 wt. %, and in a further aspect from 0.1 wt. % to 2.0 wt. % of the total weight of the personal care compositions of the present invention.

Viscosity Adjusters

The viscosity adjusters that can be included in the topically acceptable personal care composition include isopropyl alcohol, ethanol, sorbitol, propylene glycol, diethylene glycol, triethylene glycol, dimethyl ether, butylene glycol, and the like, and mixtures thereof. The viscosity adjusters, if utilized, comprise from 0.1 wt. % to 60 wt. % of the total weight of the personal care composition. In another aspect the amount ranges from 1 wt. % to 40 wt. %, and in a further aspect from 5 wt. % to 20 wt. % of the total weight of the personal care compositions of the present invention.

Other optional components can be used in order to maintain and enhance the properties of the personal care compositions of the present invention. Such optional components include various polymers for aiding the film-forming properties and substantivity of the compositions, antioxidants, and agents suitable for aesthetic purposes, such as fragrances, pigments, colorings, glitter, pearlizing agents, and the like.

The inverse emulsion polymers described herein have better electrolyte stability and are more efficient than those of the prior art, i.e., less polymer is needed to provide compositions desired by the personal care industry. The improved personal care compositions of the present invention also are less sensitive to shear than those of the prior art, resulting in longer shelf and use life of the final personal care compositions, excellent formulation aesthetics (skin feel, residue, moisturizing, emolliency, rub-in, absorption and adsorption characteristics, and the like).

It is to be noted that while varying ranges of the component ingredients of the present personal care composition have been disclosed, the amount of each component selected for the personal care composition will depend on the desired end-use and properties of the composition. In all instances the amount of each ingredient employed in the personal care composition will be selected from the disclosed range such that the summation of ingredient amounts total 100 wt. %.

The following examples are presented for the purpose of illustrating various aspects of the invention disclosed herein in greater detail. The examples, however, are not to be construed as limiting the claimed invention herein in any manner.

## EXAMPLES

### Ingredient Amounts

All ingredient amounts in the following examples are listed as weight percentages based on the total weight of the respective personal care composition. Polymer concentrations used in the following examples are an "as supplied" (polymer and carrier) weight percentage amount of polymer based on the total weight of the respective personal care composition unless specified as "weight percent based on polymer solids". In the case of weight percent based on polymer solids, this is defined as the amount of polymer added based on the total weight of monomer in the reaction mixture.

## Test Methods

Viscosity

Brookfield viscosity was measured using a Brookfield DVII+ viscometer with the appropriate RV spindle and speed indicated in each example below, viscosity readings were taken after the sample was subjected to three minutes of testing using the Brookfield DVII+ Viscometer (Brookfield Engineering Labs., Inc.). As is well-known by those of ordinary skill in the art, the selection of spindle size and speed to obtain an accurate viscosity reading is well-known (See "More Solutions to Sticky Problems", Brookfield Engineering Labs, Inc., p. 9, copyright, April 2000 and available on the Internet at http://www.brookfieldengineering.com/support/viscosity).

Yield value was calculated using the formula (Va - Vb)/100, wherein Va is the viscosity at the slowest available viscometer speed, and Vb is the viscosity at the next-to-slowest viscometer speed. See "More Solutions to Sticky Problems", p. 21, *supra.*

Freeze/Thaw Determination

Freeze/thaw (F/T) performance was tested by placing a sample in a freezer for 24 hours, removing the sample from the freezer and allowing it to thaw for 24 hours, and then repeating the freeze/thaw cycle four more times. A "pass" was regarded as no phase separation of the sample after five cycles as determined by visual inspection.

Measurement of Electrolyte or Salt Tolerance

The electrolyte tolerance of the inverse emulsion polymers utilized in the personal care compositions of the present invention is expressed in terms of viscosity maintained after a thickened sample of inverse emulsion polymer in water has been exposed to a known amount of salt. An initial sample is prepared by weighing an appropriate amount of polymer solids (based on neat polymer) and dispersing the polymer in deionized water to give a 1 wt. % w/w polymer dispersion in water. The dispersion is then mixed using a Heidolph High Torque Mixer (Fisher Scientific 2000/2001 Catalog No. 14-500) outfitted with a 3-bladed marine propeller mixing blade (5.08 cm (2 in.) diameter, Cole-Parmer Instrument Co. 2001/2002 Cat. No. U-04553-64) mounted on an 45.72 cm (18 in.) blade shaft (Cole-Parmer Instrument Co. 2001/2002 Cat. No. U-04553-55) at 800-1200 rpm for 20 minutes. At the completion of the mixing cycle, the initial viscosity of the sample is measured by placing the mixed sample on a Brookfield DVII+ Viscometer with a speed setting of 20 rpm and the appropriate RV spindle (No. 6 spindle). The initial viscosity reading is taken at 3 minutes into the measurement and recorded. Upon the determination of the initial viscosity, a quantity of monovalent salt (NaCl) or divalent salt ($MgCl_2$) sufficient to give a salt concentration of 0.1 % or (depending on the test parameter utilized) w/w (based on the total weight of the initial sample) is added to the sample under hand stirring for 5 minutes. The salt containing sample placed on the Brookfield DVII+ Viscometer set at 20 rpm (no. 4 spindle). The final viscosity reading is taken 3 minutes into the measurement and recorded. Percent viscosity maintained is calculated using the following equation:

$$\frac{\text{final viscosity}}{\text{initial viscosity}} \times 100 = \%\ \text{viscosity maintained}$$

**Example 1 - Preparation of Hand and Body Lotion**

All ingredients are listed in Table 1, together with weight percentage, function, trade name (if available) and supplier (if available) of each ingredient. Part A ingredients were combined by dissolving disodium EDTA (disodium ethylene diamine tetraacetic acid) in water, mixing until uniform, and heating the Part A mixture to 45°C. All four Part B ingredients were mixed in a separate vessel, and heated with mixing until the paraben compounds were dissolved. The Part B mixture was added with continuous mixing to the Part A mixture and heated with mixing to 65°C. Part C ingredients then were added to the mixture and mixed until the temperature fell to 40°C. Acrylic Acid Copolymer was added and mixing continued for two hours. All mixing was moderate, i.e., 800-1200 rpm using a Heidolph mixer with a marine propeller mixing blade.

Table 1 -

| Hand and Body Lotion | | | | |
|---|---|---|---|---|
| | **Ingredient** | **Wt. %** | **Function** | **Trade Name (Supplier)** |
| **Part A** | Deionized Water | 83.75 | Diluent | |
| | Disodium EDTA | 0.10 | Chelation | (Dow Chemical) |
| **Part B** | Propylene Glycol | 0.80 | Humectant | |
| | Glycerin | 5.00 | Humectant | |
| | Methylparaben | 0.10 | Preservative | |
| | Propylparaben | 0.10 | Preservative | |
| **Part C** | Mineral Oil | 4.00 | Moisture Barrier | Drakeol™ 21 (Penreco) |
| | Stearic Acid | 2.00 | Moisture Barrier | Hystren® 5016 NF 3X Pressed |
| | Glycol Stearate | 1.50 | Opacifier | |
| | Cetyl Acetate/ Acetylated Lanolin Alcohol | 0.50 | Moisture Barrier | Acetulan® (Amerchol) |
| | Glyceryl Stearate | 0.50 | Emulsifier | |
| | Cetyl Alcohol | 0.20 | Moisture Barrier | |
| | Dimethicone | 0.50 | Lubricant | Dow Corning® 200 fluid, 100 cs. (Dow Corning) |
| | Acrylates / Acrylamide / $C_{12-30}$ polyoxyalkylene (meth)acrylate Crosspolymer (and) Mineral Oil (and) Polysorbate 85 | 1.13* | Rheology modifier / Stabilizer | *Novemer™ EC-1 polymer |

* Novemer™ EC-1 polymer used as is in the formulation

The hand and body lotion was tested as follows. All viscosities were measured after 3 minutes of stirring using a #6 spindle at 20 rpm. Viscosities and freeze/thaw (F/T) test results were obtained as set forth in Table 2.

Table 2 -

| Hand and Body Lotion | | |
|---|---|---|
| | **Room Temperature** | **45° C Stability** |
| | **Viscosity (mPa·sec)** | **Viscosity (mPa·sec)** |
| Initial | 13,000 | 13,000 |
| 2 Weeks | 12,720 | 12,400 |
| 4 Weeks | 12,020 | 12,120 |
| 6 Weeks | 12,500 | 11,760 |
| 8 Weeks | 12,560 | 11,460 |
| 10 Weeks | 12,460 | 11,020 |
| 12 Weeks | 12,280 | 10,960 |
| Formulation pH: 6.65<br>5 Cycle Freeze/ Thaw: Pass | | |

**Example 2 - Preparation of Body Lotion Containing Sodium PCA**

All ingredients are listed in Table 3, together with weight percentage, function, trade name (if available) and supplier (if available) of each ingredient. Part A ingredients were combined heating to 35°C. Part B ingredients were mixed in a separate vessel, and heated with mixing until cocoa butter was dissolved. The Part A mixture was added with continuous mixing to the Part B mixture. Part C ingredients then were added to the mixture and mixing was continued until the finished product was smooth and homogeneous. All mixing was moderate, i.e., 800-1200 rpm using a Heidolph mixer and a marine propeller mixing blade.

Table 3 -

| Body Lotion Using Sodium PCA | | | | |
|---|---|---|---|---|
| | **Ingredient** | **Wt. %** | **Function** | **Trade Name (Supplier)** |
| **Part A** | Deionized Water | 83.10 | Diluent | |
| | Glycerin | 2.00 | Humectant | |
| **Part B** | Caprylic / Capric Triglycerides | 5.00 | Emollient | Liponate® GC (Lipo) |
| | Sunflower Seed Oil | 3.00 | Emollient | Lipovol® Sun (Lipo) |
| | Cetearyl Octanoate | 2.00 | Emollient | Crodamol® CAP |
| | Cocoa Butter | 1.50 | Emollient | Fancol™ CB USP (Fanning Corporation) |
| | Acrylates / Acrylamide / $C_{12-30}$ polyoxyalkylene (meth)acrylate Crosspolymer (and) Mineral Oil (and) Polysorbate 85 | 2.30* | Emulsifer / Rheology modifier | *Novemer™ EC-1 polymer |
| **Part C** | Fragrance | 0.20 | Fragrance | Lotion Fragrance #7960 (Belle Aire Fragrances) |
| | DMDM Hydantoin | 0.40 | Preservative | Glydant® (Lonza) |
| | Sodium Pyrrolidone Carboxylic Acid (50%) | 0.50 | Humectant | Ajidew® N-50 (Ajinomoto) |

* Novemer™ EC-1 polymer used as is in the formulation

The body lotion containing sodium PCA was tested as follows. All viscosities were measured after 3 minutes of stirring using a #6 spindle at 20 rpm. Viscosities and freeze/thaw (F/T) test results were obtained as set forth in Table 4.

Table 4 -

| Body Lotion Containing Sodium PCA | | |
|---|---|---|
| | **Room Temperature** | **45° C stability** |
| | **Viscosity (mPa·sec)** | **Viscosity (mPa·sec)** |
| Initial | 13,180 | 13,180 |
| 2 Weeks | 14,020 | 12,300 |
| 4 Weeks | 14,160 | 11,900 |
| 6 Weeks | 14,100 | 12,040 |
| 8 Weeks | 14,320 | 11,500 |

Table 4 - (continued)

| Body Lotion Containing Sodium PCA | | |
|---|---|---|
| | Room Temperature | 45° C stability |
| | Viscosity (mPa·sec) | Viscosity (mPa·sec) |
| 10 Weeks | 14,160 | 11,460 |
| 12 Weeks | 14,220 | 11,280 |
| Formulation pH: 6.68<br>5 Cycle Freeze / Thaw: Pass | | |

**Example 3 - Preparation of Sprayable Sunscreen**

All ingredients are listed in Table 5, together with weight percentage, function, trade name (if available) and supplier (if available) of each ingredient. Part A ingredients were combined and with mixing and heated to 55°C. Part B ingredients were mixed in a separate vessel, and heated to 55°C. Part A was added to Part B with mixing at 55°C and mixed until homogeneous with heat removed. Part C ingredients were added at 40°C with mixing. Part D was added at room temperature with mixing. All mixing was moderate, i.e., 800-1200 rpm using a Heidolph mixer and a marine propeller mixing blade.

Table 5 -

| Sprayable Sunscreen | | | | |
|---|---|---|---|---|
| | Ingredient | Wt. % | Function | Trade Name (Supplier) |
| Part A | Deionized water | 71.15 | Diluent | |
| | Propylene Glycol | 3.00 | Humectant | |
| Part B | Octyl Methoxycinnamate | 7.00 | UV-B Sunscreen | Heliopan® AV (H&R) |
| | Octyl Salicylate | 3.00 | UV-B Sunscreen | Octyl Salicylate (Noveon, Inc.) |
| | Avobenzone | 2.50 | UV-A Sunscreen | Parsol® 1789 (Roche) |
| | PPG-2 Myristyl Ether Propionate | 7.00 | Emollient | Crodamol® PMP (Croda) |
| | Octyl Stearate | 2.50 | Emollient | Lexol® EHS (Inolex) |
| | Acrylates / Acrylamide / $C_{12-30}$ Polyoxyalkylene (meth)acrylate Crosspolymer (and) Mineral Oil (and) Polysorbate 85 | 0.75* | Emulsifier / Stabilizer / Rheology Modifier | *Novemer™ EC-1 polymer (Noveon, Inc.) |

* Novemer™ EC-1 polymer used as is in the formulation

Table 5 - (continued)

| Sprayable Sunscreen | | | | |
|---|---|---|---|---|
| | Ingredient | Wt. % | Function | Trade Name (Supplier) |
| **Part C** | PEG-20 Almond Glycerides | 0.50 | Emulsifier | Crovol® A-40 (Croda) |
| | Cyclomethicone and Dimethiconol | 2.00 | Spread aid | Dow Corning® DC 1401 (Dow Coming) |
| | Fragrance | 0.25 | Fragrance | Twister 829 718 (Drom) |
| | DMDM Hydantoin | 0.30 | Preservative | Glydant® (Lonza) |
| **Part D** | Disodium EDTA | 0.05 | Chelating agent | Versene® $Na_2$EDTA (Dow) |

The sprayable sunscreen was tested as follows. All viscosities were measured after 3 minutes of stirring using a #6 spindle at 20 rpm. Viscosities were obtained as set forth in Table 6.

Table 6 -

| Sprayable Sunscreen | | |
|---|---|---|
| | Room Temperature | 45° C stability |
| | Viscosity (mPa·sec) | Viscosity (mPa·sec) |
| Initial | 3,385 | 3,385 |
| 2 Weeks | 3,595 | 4,365 |
| 4 Weeks | 3,830 | 4,390 |
| 6 Weeks | 3,880 | 4,500 |
| 8 Weeks | 4,020 | 4,500 |
| 10 Weeks | 3,920 | 4,580 |
| 12 Weeks | 4,000 | 4,580 |
| Formulation pH: 6.70<br>5 Cycle Freeze / Thaw: Pass | | |

**Example 4 - Day Cream with Physical Sunscreen**

All ingredients are listed in Table 7, together with weight percentage, function, trade name (if available) and supplier (if available) of each ingredient. Part A ingredients were combined with mixing and heated to 65°C. Part B ingredients were mixed in a separate vessel without adding the zinc oxide material, and heated to 65° C. Part A was added to Part B with mixing at 65°C and mixed until homogeneous with heat removed. Zinc oxide was added to the composition with agitation at 65° C. Part C ingredients were added at 40° C with mixing. All mixing was moderate, i. e., 800-1200 rpm using a Heidolph mixer and a marine propeller mixing blade.

Table 7 -

| Day Cream with Physical Sunscreen | | | | |
|---|---|---|---|---|
| | **Ingredient** | **Wt. %** | **Function** | Trade Name **(Supplier)** |
| **Part A** | Deionized water | 72.85 | Diluent | |
| | Glycerin | 8.00 | Humectant | |
| | Acrylates / Acrylamide / $C_{12-30}$polyoxyalkylene (meth)acrylate Crosspolymer (and) Mineral Oil (and) Polysorbate 85 | 1.65* | Rheology modifier / Stabilizer | *Novemer™ EC-1 polymer (Noveon, Inc.) |
| | Disodium EDTA | 0.05 | Chelating Agent | Versene® $Na_2$EDTA (Dow) |
| **Part B** | Octyl Methoxycinnamate | 5.00 | UV-B Sunscreen | Heliopan® AV (H&R) |
| | Isohexadecane | 3.00 | Emollient | Arlamol® HD (Uniqema) |
| | Isopropyl Palmitate | 1.50 | Emollient | Estol® 1517 (Uniqema) |
| | Dimethicone and Dimethiconol | 1.00 | Slip aid | Dow Corning® DC 1403 Fluid (Dow Coming) |
| | Steareth-21 | 0.35 | Emulsifier | Brij 721 (Uniqema) |
| | Steareth-2 | 0.65 | Emulsifier | Brij 72 (Uniqema) |
| | Cetearyl Alcohol | 1.00 | Opacifier | Lanette Wax® O (Cognis) |
| | Behenyl Alcohol | 0.75 | Opacifier | Lanette® 22 (Cognis) |
| | DEA Oleth-3 Phosphate | 0.50 | Emulsifier | Crodafos™ N-3 Neutral (Croda) |
| | Zinc Oxide and Dimethicone | 3.00 | UV-A block | Z-Cote™ HP1 (BASF) |
| **Part C** | DMDM Hydantoin | 0.50 | Preservative | Glydant® (Lonza) |
| | Vitamin E Acetate | 0.20 | Active | Vitamin E Acetate (BASF) |
| * Novemer™ EC-1 polymer used as commercially supplied, in the formulation | | | | |

The day cream with physical sunscreen was tested as follows. All viscosities were measured after 3 minutes of stirring using a #6 spindle at 20 rpm. Viscosities were obtained as set forth in Table 8.

Table 8 -

| Day Cream with Physical Sunscreen | | |
|---|---|---|
| | Room Temperature | 45° C stability |
| | Viscosity (mPa·sec) | Viscosity (mPa·sec) |
| Initial | 27,000 | 27,000 |
| 2 Weeks | | |
| 4 Weeks | 29,400 | 23,200 |
| 6 Weeks | 29,000 | 21,400 |
| 8 Weeks | 28,400 | 17,500 |
| 10 Weeks | 28,500 | 13,000 |
| 12 Weeks | 29,000 | 13,000 |
| Formulation pH: 8.70<br>5 Cycle Freeze / Thaw: Pass | | |

**Example 5 - Sprayable After-Sun Moisturizer**

All ingredients are listed in Table 9, together with weight percentage, function, trade name (if available) and supplier (if available) of each ingredient. Part A ingredients were combined with mixing for 5 minutes at 1100 rpm. Part B is slowly added to Part A with rapid agitation. Part C ingredients were slowly added with mixing. Mixing was continued until homogeneous. All mixing was moderate, i.e., 800-1200 rpm using a Heidolph mixer and a marine propeller mixing blade.

Table 9 -

| Sprayable After-Sun Moisturizer | | | | |
|---|---|---|---|---|
| | Ingredient | Wt. % | Function | Trade Name (Supplier) |
| Part A | Deionized Water | 85.15 | Diluent | |
| | Propylene Glycol and Diazolidinyl Urea and Methylparaben and Propylparaben | 0.80 | Preservative | Germaben II™ (Sutton) |
| | Cetearyl Ethylhexanoate | 1.50 | Emollient | Crodamol® CAP (Croda) |
| | Isopropyl Palmitate | 3.00 | Emollient | Estol® 1517 (Uniqema) |
| | Safflower Seed Oil | 4.00 | Emollient | Lipovol® SAF (Lipo) |
| | Cyclomethicone and Dimethiconol | 0.50 | Slip aid | Dow Corning® DC 1401 Fluid (Dow Corning) |
| | PEG-7 Dimethicone Isostearate | 0.85 | Emulsifier | Ultrasil™ DW-18 (Noveon, Inc.) |

Table 9 - (continued)

| Sprayable After-Sun Moisturizer | | | | |
|---|---|---|---|---|
| | **Ingredient** | **Wt. %** | **Function** | **Trade Name (Supplier)** |
| **Part B** | Acrylates / Acrylamide / $C_{12-30}$ Polyoxyalkylene (meth)acrylate Crosspolymer (and) Mineral Oil (and) Polysorbate 85 | 2.00* | Emulsifier / Rheology modifier | *Novemer™ EC-1 polymer (Noveon, Inc.) |
| **Part C** | Aloe Barbadensis Leaf Juice | 1.50 | Botanical Active | Aloe Vera Gel Concentrate 40:1 (Aloe Corp) |
| | Water and Hydrolyzed Coralina Officinalis (Coral Seaweed) | 0.50 | Humectant | Oligophycocorail (Secma) |
| | Fragrance | 0.20 | Fragrance | Dewfruit Bouquet J-7418-B (Bell Flavors and Fragrances Inc.) |

* Novemer™ EC-1 polymer used as commercially supplied, in the formulation

The sprayable after-sun moisturizer was tested as follows. All viscosities were measured after 3 minutes of stirring using a #6 spindle at 20 rpm. Viscosities were obtained as set forth in Table 10.

Table 10 -

| Sprayable After-Sun Moisturizer | | |
|---|---|---|
| | **Room Temperature** | **45° C stability** |
| | **Viscosity (mPa·sec)** | **Viscosity (mPa·sec)** |
| Initial | 2,280 | 2,280 |
| 2 Weeks | 2,195 | 2,255 |
| 4 Weeks | 2,225 | 2,340 |
| 6 Weeks | 2,295 | 2,420 |
| 8 Weeks | 2,280 | 2,370 |
| 10 Weeks | 2,260 | 2,310 |
| 12 Weeks | 2,300 | 2,185 |
| Formulation pH: 6.35<br>3 Cycle Freeze / Thaw: Pass | | |

**Example 6 - Facial Moisturizer**

All ingredients are listed in Table 11, together with weight percentage, function, trade name (if available) and supplier (if available) of each ingredient. Part A ingredients were combined with mixing and heated to 65°C. Part B ingredients were combined and heated to 65°C with mixing (polymer was added after reaching temperature of 65°C). Part A was added to Part B with rapid agitation. Part C ingredients were added in order when composition temperature was below 40°C. All mixing was moderate, i.e., 800-1200 rpm using a Heidolph mixer and a marine propeller mixing blade.

Table 11 -

| Facial Moisturizer | | | | |
|---|---|---|---|---|
| | **Ingredient** | **Wt. %** | **Function** | **Trade Name (Supplier)** |
| **Part A** | Deionized Water | 83.60 | Diluent | |
| | Guar Hydroxypropyl-trimonium chloride | 0.10 | Skin conditioner | Hi-Care® 1000 (Rhodia) |
| | Glycerin | 3.00 | Humectant | |
| **Part B** | Octyl Stearate | 3.00 | Emollient | Lexol® EHS (Inolex) |
| | Triticum Vulgare (Wheat) Germ Oil | 2.00 | Emollient | Cropure® Wheatgerm (Croda) |
| | Cetyl Alcohol | 1.00 | Opacifier | Lanette® 16 NF (Cognis) |
| | Octyl Methoxycinnamate | 2.00 | UV-B Sunscreen | Heliopan® AV (H&R) |
| | Glyceryl Stearate and PEG-100 Stearate | 1.50 | Emulsifier | Aralacel® 165 (Uniqema) |
| | Acrylates / Acrylamide / $C_{12-30}$ Polyoxyalkylene (meth)acrylate Crosspolymer (and) Mineral Oil (and) Polysorbate 85 | 1.50* | Emulsifier / Rheology modifier | *Novemer™ EC-1 polymer (Noveon, Inc.) |
| **Part C** | Propylene Glycol and Cucumis Sativus (Cucumber) Fruit Extract | 1.00 | Botanical Active | Vegetol® Cp GR 049 Hydro (Gattefosse) |
| | Propylene Glycol and Fucus Vesiculosus (Seaweed) Extract | 1.00 | Botanical Active | Vegetol® Algues CB 4136 Hydro (Gattefosse) |
| | Phenoxyethanol and Methylparaben and Butylparaben and Ethylparaben and Propylparaben | 0.30 | Preservative | Phenonip® (Clariant) |

* Novemer™ EC-1 polymer used as commercially supplied, in the formulation

The facial moisturizer was tested as follows. All viscosities were measured after 3 minutes of stirring using a #6 spindle at 20 rpm. Viscosities were obtained as set forth in Table 12.

Table 12 -

| Facial Moisturizer | | |
|---|---|---|
| | Room Temperature | 45° C stability |
| | Viscosity (mPa·sec) | Viscosity (mPa·sec) |
| Initial | 33,100 | 33,100 |
| 2 Weeks | 35,900 | 29,700 |
| 4 Weeks | 35,750 | 29,000 |
| 6 Weeks | N/A | 28,000 |
| 8 Weeks | N/A | 24,000 |
| 10 Weeks | N/A | 24,250 |
| 12 Weeks | N/A | 24,000 |
| Formulation pH: 6.84<br>5 Cycle Freeze / Thaw: Pass | | |

**Example 7 - Night Cream with Moisturizing Actives**

All ingredients are listed in Table 13, together with weight percentage, function, trade name (if available) and supplier (if available) of each ingredient. Part A ingredients were combined with mixing and heated to 70°C. Part B ingredients were combined and heated to 70°C with mixing (polymer was added after reaching temperature of 70°C). Part A was added to Part B with rapid agitation. Part C ingredients were added in order when composition temperature was below 40°C. All mixing was moderate, i.e., 800-1200 rpm using a Heidolph mixer and a marine propeller mixing blade.

Table 13 -

| Night Cream with Moisturizing Actives | | | | |
|---|---|---|---|---|
| | Ingredient | Wt. % | Function | Trade Name (Supplier) |
| Part A | Deionized Water | 76.95 | Diluent | |
| | Glycerin | 3.00 | Humectant | |

Table 13 - (continued)

| Night Cream with Moisturizing Actives | | | | |
|---|---|---|---|---|
| | **Ingredient** | **Wt. %** | **Function** | **Trade Name (Supplier)** |
| **Part B** | Hydrogenated Polyisobutene | 5.00 | Emollient | Panalane® L-14E (Lipo) |
| | Caprylic / Capric Triglycerides | 3.50 | Emollient | Liponate® GC (Lipo) |
| | Isohexadecane | 3.00 | Emollient | Arlamol® HD (Uniqema)) |
| | Evening Primrose Oil | 2.00 | Emollient | Evening Primrose Oil (Croda) |
| | Glyceryl Stearate and PEG-100 Stearate | 2.00 | Emulsifier | Aralacel® 165 (Uniqema) |
| | Cetearyl Alcohol / Ceteareth-20 | 1.20 | Opacifier | Emulgade® 1000NI (Cognis) |
| | Acrylates / Acrylamide / $C_{12-30}$ Polyoxyalkylene (meth)acrylate Crosspolymer (and) Mineral Oil (and) Polysorbate 85 | 1.55* | Emulsifier / Rheology modifier | *Novemer™ EC-1 polymer (Noveon, Inc.) |
| **Part C** | Hydrolyzed Ellastin | 1.00 | Active | Crolastin® (Croda) |
| | Hydrolyzed Protein / Hyaluronic Acid | 0.50 | Active | Cromoist® HYA (Croda) |
| | Phenoxyethanol and Methylparaben and Butylparaben and Ethylparaben and Propylparaben | 0.30 | Preservative | Phenonip® (Clariant) |

* Novemer™ EC-1 polymer used as commercially supplied, in the formulation

The night cream with moisturizing actives was tested as follows. All viscosities were measured after 3 minutes of stirring using a #6 spindle at 20 rpm. Viscosities were obtained as set forth in Table 14.

Table 14 -

| Night Cream with Moisturizing Actives | | |
|---|---|---|
| | **Room Temperature** | **45° C stability** |
| | **Viscosity (mPa·sec)** | **Viscosity (mPa·sec)** |
| Initial | 30,750 | 30,750 |
| 2 Weeks | 28,400 | 29,100 |

Table 14 - (continued)

| Night Cream with Moisturizing Actives | | |
|---|---|---|
| | Room Temperature | 45° C stability |
| | Viscosity (mPa·sec) | Viscosity (mPa·sec) |
| 4 Weeks | 32,000 | 26,450 |
| 6 Weeks | 33,000 | 26,750 |
| 8 Weeks | 33,600 | 23,100 |
| 10 Weeks | 33,100 | 23,200 |
| 12 Weeks | 30,100 | 23,100 |
| Formulation pH: 6.60<br>5 Cycle Freeze / Thaw: Pass | | |

**Example 8 - Comparative Thickening Performance**

Example 8 demonstrates the superior thickening ability of the inverse emulsion polymer used in the personal care compositions of the present invention, as compared to two commercially available thickeners. Samples were prepared by mixing the indicated weight percentages based on polymer solids into deionized water and allowing to mix for 20 minutes at 800-1200 rpm using a Heidolph mixer with a marine propeller blade and thereafter measuring viscosities using # 3 to #6 spindles at 20 rpm. Test data is set forth in Table 15.

Table 15

| Comparative Thickening Performance | | | | | |
|---|---|---|---|---|---|
| Polymer Solids | 0.25 Wt. % | 0.50 Wt. % | 0.75 Wt. % | 1.00 Wt. % | 1.25 Wt. % |
| Sepigel® 305[1] | 1,400 (mPa·sec) | 11,120 (mPa·sec) | 20,350 (mPa·sec) | 26,200 (mPa·sec) | 30,900 (mPa·sec) |
| Salcare® SC 91[2] | 1,475 (mPa·sec) | 6,800 (mPa·sec) | 12,700 (mPa·sec) | 20,750 (mPa·sec) | 27,350 (mPa·sec) |
| Novemer ™ EC-1 polymer[3] | 7,100 (mPa·sec) | 15,600 (mPa·sec) | 22,400 (mPa·sec) | 26,200 (mPa·sec) | 32,400 (mPa·sec) |

[1] Commercially available from Seppic.
[2] Commercially available from Ciba Specialty Chemicals Corporation.
[3] Commercially available from Noveon, Inc.

**Example 9 - Comparative Yield Values**

Example 9 demonstrates that the inverse emulsion polymer used in the personal care compositions of the present invention provides superior yield values, as compared to two commercially available thickeners. Samples were prepared by mixing the indicated weight percentages based on polymer solids into deionized water and allowing to mix for 20 minutes at 800-1200 rpm using a Heidolph mixer with a marine propeller blade and thereafter measuring viscosities using #3 to #6 spindles at 1.0 rpm and at 0.5 rpm, and using the following equation. Test data is set forth in Table 16.

$$\text{Yield Value} = (\text{Viscosity at 0.5 rpm} - \text{Viscosity at 1.0 rpm}) / 100$$

Table 16 -

| Comparative Yield Values | | | | | |
|---|---|---|---|---|---|
| **Polymer Solids** | **0.25 Wt. %** | **0.50 Wt. %** | **0.75 Wt. %** | **1.00 Wt. %** | **1.25 Wt. %** |
| Sepigel® 305[1] | 64 | 850 | 1,492 | 2,216 | 2,300 |
| Salcare® SC91[2] | 21 | 218 | 572 | 1,160 | 1,480 |
| Novemer™ EC-1 polymer[3] | 388 | 1,340 | 1,920 | 2,270 | 2,670 |

[1] Commercially available from Seppic
[2] Commercially available from Ciba Specialty Chemicals Corporation
[3] Commercially available from Noveon, Inc.

**Example 10 - Monovalent Electrolyte Tolerance**

Example 10 demonstrates that the inverse emulsion polymer used in the personal care compositions of the present invention provides superior electrolyte tolerance (indicated by higher viscosity), as compared to two commercially available thickeners.

Polymer samples were prepared by adding them at a concentration of 1.0 wt. % based on polymer solids into deionized water. The sample was mixed using a Heidolph mixer and a marine propeller mixing blade. Mixing was done at 800-1200 rpm for 20 minutes. Sample viscosities were then measured using a Brookfield DVII+ Viscometer set at a speed of 20 rpm using RV spindles. Viscosities were recorded at three (3) minutes into the measurement. Once viscosities were measured, known quantities of monovalent salt, in this case sodium chloride, or in the case of divalent salt, magnesium chloride was added to the sample with hand mixing for 5 minutes. After each addition of salt, a viscosity reading was taken and listed in the following table, and thereafter measuring viscosities using Brookfield #2 to 6 spindles at 20 rpm. Test data is set forth in Table 17.

Table 17 -

| Monovalent Electrolyte Tolerance | | | | | | |
|---|---|---|---|---|---|---|
| | **NaCl 0.0 Wt. %** | **NaCl 0.10 Wt. %** | **NaCl 0.25 Wt. %** | **NaCl 0.50 Wt. %** | **NaCl 0.75 Wt. %** | **NaCl 1.00 Wt. %** |
| Sepigel® 305[1] | 28,100 (mPa·sec) | 4,140 (mPa·sec) | 550 (mPa·sec) | 195 (mPa·sec) | 42 (mPa·sec) | 30 (mPa·sec) |
| Salcare® SC91[2] | 20,750 (mPa·sec) | 7,340 (mPa·sec) | 1,685 (mPa·sec) | 460 (mPa·sec) | 200 (mPa·sec) | 114 (mPa·sec) |
| Novemer™ EC-1 polymer[3] | 26,600 (mPa·sec) | 20,500 (mPa·sec) | 14,600 (mPa·sec) | 6,880 (mPa·sec) | 4,050 (mPa·sec) | 2,300 (mPa·sec) |

[1] Commercially available from Seppic
[2] Commercially available from Ciba Specialty Chemicals Corporation
[3] Commercially available from Noveon, Inc.

Table 18 -

| Percent Viscosity Maintained with Addition of Monovalent Electrolyte | | | | | | |
|---|---|---|---|---|---|---|
| | **NaCl 0.0 Wt. %** | **NaCl 0.10 Wt. %** | **NaCl 0.25 Wt. %** | **NaCl 0.50 Wt. %** | **NaCl 0.75 Wt. %** | **NaCl 1.00 Wt. %** |
| Sepigel® 305[1] | 28,100 (mPa·sec) | 14.73% | 1.96% | 0.69% | 0.15% | 0.11% |
| Salcare® SC91[2] | 20,750 (mPa·sec) | 35.37% | 8.12% | 2.22% | 0.96% | 0.55% |

[1] Commercially available from Seppic
[2] Commercially available from Ciba Specialty Chemicals Corporation

Table 18 - (continued)

| Percent Viscosity Maintained with Addition of Monovalent Electrolyte | | | | | | |
|---|---|---|---|---|---|---|
| | **NaCl 0.0 Wt. %** | **NaCl 0.10 Wt. %** | **NaCl 0.25 Wt. %** | **NaCl 0.50 Wt. %** | **NaCl 0.75 Wt. %** | **NaCl 1.00 Wt. %** |
| Novemer™ EC-1 polymer[3] | 26,600 (mPa·sec) | 77.07% | 54.89% | 25.86% | 15.23% | 8.65% |

[3] Commercially available from Noveon, Inc.

**Example 11 - Electrolyte Tolerance at Higher Polymer Solids Concentrations**

Example 11 demonstrates that the inverse emulsion polymer used in the personal care compositions of the present invention provides superior electrolyte tolerance (indicated by higher viscosity), as compared to commercially available thickeners. Polymer samples were prepared by mixing them at a concentration of 2.0 wt. % based on polymer solids into deionized water. The sample was mixed using a Heidolph mixer and a marine propeller mixing blade. Mixing was done at 800-1200 rpm for 20 minutes. Sample viscosities were then measured using a Brookfield DVII+ Viscometer set at a speed of 20 rpm using RV spindles. Viscosities were recorded at three (3) minutes into the measurement. Once viscosities were measured, known quantities of monovalent salt, in this case sodium chloride, or in the case of divalent salt, magnesium chloride was added to the sample with hand mixing for 5 minutes. After each addition of salt, a viscosity reading was taken and recorded as listed in the following table, and thereafter measuring viscosities using Brookfield #2 to 6 spindles at 20 rpm. Test data is set forth in Table 19.

Table 19 -

| 2.0 wt. % Polymer with Sodium Chloride Added | | | | | | | | |
|---|---|---|---|---|---|---|---|---|
| | Polymer Solids | Wt. % Sodium chloride added to mucilage | | | | | | |
| | | 0.0 | 0.5 | 1.0 | 2.0 | 3.0 | 4.0 | 5.0 |
| Novemer™ EC-1 Polymer | 2 wt. % | 68,800 (mPa·sec) | 68,000 (mPa·sec) | 61,200 (mPa·sec) | 46,000 (mPa·sec) | 33,000 (mPa·sec) | 24,450 (mPa·sec) | 20,000 (mPa·sec) |
| Salcare® SC91 | 2 wt. % | 56,000 (mPa·sec) | 15,560 (mPa·sec) | 3,990 (mPa·sec) | Fail | Fail | Fail | Fail |
| Sepigel® 305 | 2 wt. % | 52,800 (mPa·sec) | 6,290 (mPa·sec) | 1,475 (mPa·sec) | Fail | Fail | Fail | Fail |
| Simulgel® NS | 2 wt. % | 53,200 (mPa·sec) | 8,650 (mPa·sec) | 2,315 (mPa·sec) | Fail | Fail | Fail | Fail |
| **Fail:** No substantial viscosity, or sample separates | | | | | | | | |

**Example 12 - Comparative Yield Value in the Presence of Electrolyte**

Example 12 demonstrates that the inverse emulsion polymer used in the personal care compositions of the present invention provides superior yield values in the presence of electrolyte, as compared to commercially available thickeners. Samples were prepared by mixing them at the indicated weight percentages into deionized water and thereafter measuring viscosities using #3 to 6 spindles at 1.0 rpm and at 0.5 rpm, and using the following equation. Test data is set forth in Table 20.

Yield Value = (Viscosity at 0.5 rpm - Viscosity at 1.0 rpm) / 100

Table 20 -

| Comparative Yield Value in the Presence of Electrolyte | | | | | | | | |
|---|---|---|---|---|---|---|---|---|
| | | Wt. % Sodium Chloride Added to Mucilage | | | | | | |
| | Polymer Solids | 0.0 | 0.5 | 1.0 | 2.0 | 3.0 | 4.0 | 5.0 |
| Novemer™ EC-1 Polymer | 2 wt. % | 5,210 | 5,740 | 4,200 | 3,230 | 2,830 | 2,870 | 2,240 |
| Salcare® SC91 | 2 wt. % | 3,550 | 874 | 134 | Fail | Fail | Fail | Fail |
| Sepigel® 305 | 2 wt. % | 3,550 | 325 | NA | Fail | Fail | Fail | Fail |
| Simulgel® NS | 2 wt. % | 3,770 | 598 | 115 | Fail | Fail | Fail | Fail |
| **Fail:** No substantial viscosity, or sample separates | | | | | | | | |

**Example 13 - Divalent Electrolyte Tolerance**

Example 13 demonstrates that the inverse emulsion polymer used in the personal care compositions of the present invention provides superior electrolyte tolerance (indicated by higher viscosity), as compared to two commercially available thickeners. Polymer samples were prepared by mixing them at a concentration of 1.0 wt. % based on polymer solids into deionized water. The sample was mixed using a Heidolph mixer and a marine propeller mixing blade. Mixing was done at 800-1200 rpm for 20 minutes. Sample viscosities were then measured using a Brookfield DVII+ Viscometer set at a speed of 20 rpm using RV spindles. Viscosities were recorded at three (3) minutes into the measurement. Once viscosities were measured, known quantities of divalent salt, magnesium chloride, was added to the sample with hand mixing for 5 minutes. After each addition of salt, a viscosity reading was taken and recorded in the following table, and thereafter measuring viscosities using Brookfield #2 to 6 spindles at 20 rpm. Test data is set forth in Table 21.

Table 21 -

| Divalent Electrolyte Tolerance | | | | | |
|---|---|---|---|---|---|
| Wt. % Magnesium chloride added to mucilage | | | | | |
| | Polymer Solids | 0.0 | 0.10 | 0.25 | 0.50 |
| Novemer™ EC-1 Polymer | 1 wt % | 28,250 (mPa·sec) | 21,450 (mPa·sec) | 14,360 (mPa·sec) | 3,250 (mPa·sec) |
| Salcare® SC91 | 1 wt. % | 27,950 (mPa·sec) | Fail | Fail | Fail |
| Sepigel® 305 | 1 wt. % | 19,300 (mPa·sec) | Fail | Fail | Fail |
| **Fail:** No substantial viscosity, or sample separates | | | | | |

**Example 14 - Emulsifying Properties**

Example 14 demonstrates that the inverse emulsion polymer used in the personal care compositions of the present invention is capable of emulsifying various oils and commonly used non-aqueous ingredients. Samples were prepared by adding 1.0 wt. % based on polymer solids to either the oil phase or the water phase prior to phasing the

two phases together, mixing was done at 800-1200 rpm for 20 minutes using a Heidolph mixer with a marine propeller mixing blade. Formulations are listed in Table 22. Overall emulsion stabilities were evaluated at 45° C for 12 weeks and viscosities were measured using Brookfield #2 to 6 spindles at 20 rpm. Test data is set forth in Table 23.

Table 22 -

| Formulations using Novemer™ EC-1 Polymer as Emulsifier | | | | | | | | | | | | | | |
|---|---|---|---|---|---|---|---|---|---|---|---|---|---|---|
| **Materials** | **Supplier / Trade Name** | **82C** | **82D** | **82E** | **82F** | **82H** | **82I** | **67F** | **67A** | **65B** | **65D** | **65F** | **84A** | **84B** |
| Deionized water | | 86.30 | 86.30 | 86.30 | 86.30 | 86.30 | 86.30 | 71.30 | 71.30 | 71.30 | 71.30 | 71.30 | 86.30 | 86.30 |
| Acrylates / Acrylamide / $C_{12-30}$ Polyoxyalkylene (meth)acrylate Crosspolymer (and) Mineral Oil (and) Polysorbate 85 | Novemer™ EC-1 polymer (Noveon, Inc.) | 3.70 | 3.70 | 3.70 | 3.70 | 3.70 | 3.70 | 3.70 | 3.70 | 3.70 | 3.70 | 3.70 | 3.70 | 3.70 |
| Isopropyl Palmitate | Estol® 1517/ Uniqema | | | | | | | | | | | | 10.00 | |
| Isopropyl Myristate | Liponate® IPM/Lipo | | | | | | | | | | | | | 10.00 |
| Caprylic/ capric Triglycerides | Crodamol® GTCC / Croda | 10.00 | | | | | | | | | | | | |
| Dimethicone | DC® 200 (100 cSt)/ Dow Coming | | 10.00 | | | | | | | | | | | |
| Cyclo-methicone | DC® 345 Dow Coming | | | 10.00 | | | | | | | | | | |
| Mineral Oil | Drakeol® 21/ Penreco | | | | 10.00 | | | | | | | | | |
| Isohexadecane | Arlamol® HD/ Uniqema | | | | | | | | | | | | | |
| Hydrogenated Polyisobutene | Panalane® L14-E/ Lipo | | | | | 10.00 | | | | | | | | |
| Octylmethoxy cinnamate | Heliopan® AV / H&R | | | | | | 10.0 0 | | | | | | | |
| Hydrogenated Castor oil | | | | | | | | 25.00 | | | | | | |
| Sunflower | Lipovol® | | | | | | | | 25.00 | | | | | |

Table 22 - (continued)

| Formulations using Novemer™ EC-1 Polymer as Emulsifier | | | | | | | | | | | | | | |
|---|---|---|---|---|---|---|---|---|---|---|---|---|---|---|
| **Materials** | **Supplier / Trade Name** | **82C** | **82D** | **82E** | **82F** | **82H** | **82I** | **67F** | **67A** | **65B** | **65D** | **65F** | **84A** | **84B** |
| Seed Oil | Sun / Lipo | | | | | | | | | | | | | |
| C12-14 Alkyl Benzoate | Finsolv® TN / Finetex | | | | | | | | | 25.00 | | | | |
| Cetearyl Octanoate | Crodamol® CAP/ Croda | | | | | | | | | | 25.00 | | | |
| PPG-2 Myristyl Ether Propionate | Crodamol® PMP / Croda | | | | | | | | | | | 25.00 | | |

Stability data of the emulsions made in Table 22 are found in Table 23. Viscosity measurements were taken on samples for 12 weeks at 45°C. Viscosity was measured using a Brookfield Viscometer DVII++. Measurements were taken at 20 rpm using RV spindles, units are mPa·s.

Table 23 -

| Emulsion Stability Data, mPa·s | | | | | | | | | | | | | |
|---|---|---|---|---|---|---|---|---|---|---|---|---|---|
| **Sample** | **Initial** | **2 weeks** | | **4 weeks** | | **6 weeks** | | **8 weeks** | | **10 weeks** | | **12 weeks** | |
| # | | R/T | 45°C | R/T | 45°C | R/T | 45°C | R/T | 45°C | R/T | 45°C | R/T | 45°C |
| 82C | 30,800 | 30,400 | 29,050 | 29,700 | 27,650 | 28,850 | 27,050 | 30,450 | 26,500 | 29,850 | 16,900 | 30,600 | 23,750 |
| 82D | 11,900 | 9,740 | 7,300 | 9,460 | 8,020 | 8,160 | 8,500 | 9,700 | 8,000 | 11,800 | 9,600 | 9,920 | 9,500 |
| 82E | 3,320 | 2,460 | 3,940 | 3,400 | 1,940 | 4,060 | 1,920 | 3,080 | 3,040 | 3,020 | 1,980 | 2,940 | 1,740 |
| 82F | 9,500 | 9,000 | 8,800 | 9,150 | 7,850 | 9,960 | 8,600 | 9,280 | 8,260 | 10,260 | 7,840 | 10,400 | 8,380 |
| 82H | 7,600 | 8,400 | 5,760 | 9,140 | 6,200 | 8,540 | 8,700 | 9,640 | 7,360 | 8,500 | 7,680 | 10,860 | 7,600 |
| 82I | 32,100 | 30,050 | 31,900 | 33,100 | 31,150 | 33,300 | 31,050 | 33,950 | 30,950 | 34,500 | 29,550 | 35,850 | 29,400 |
| 67F | 27,900 | N/A | 33,000 | N/A | 30,800 | N/A | 28,500 | N/A | N/A | N/A | N/A | N/A | 22,500 |
| 67A | 35,400 | N/A | 32,500 | N/A | 25,900 | N/A | 21,500 | N/A | N/A | N/A | N/A | N/A | 13,000 |
| 65B | 37,300 | N/A | 38,300 | N/A | 35,200 | N/A | 34,050 | N/A | N/A | N/A | N/A | N/A | 28,300 |
| 65D | 10,600 | N/A | 3,700 | N/A | 3,800 | N/A | 4,900 | N/A | N/A | N/A | N/A | N/A | 4,650 |
| 65F | 19,300 | N/A | 24,150 | N/A | 14,350 | N/A | 10,000 | N/A | N/A | N/A | N/A | N/A | 4,800 |
| 84A | 28,250 | 29,600 | 28,400 | 28,500 | 27,200 | 29,350 | 26,300 | 29,900 | 25,200 | 29,650 | 24,150 | 30,400 | 22,650 |
| 84B | 28,650 | 28,550 | 23,450 | 28,300 | 25,000 | 29,800 | 19,050 | 28,750 | 19,250 | 24,950 | 16,550 | 29,300 | 14,600 |

All emulsions in Table 22 appeared homogeneous without separation over the 12 week stability study at 45°C.

**Example 15 - Emulsification Properties Demonstrated**

Example 15 demonstrates that the inverse emulsion polymer used in the personal care compositions of the present invention is capable of emulsifying various oils and commonly used non-aqueous ingredients. Samples were prepared by adding approximately 0.5 wt. % by weight polymer solids to either the oil phase or the water phase, prior to phasing the two phases together, or to the phased emulsion. All mixing was done using a Heidolph mixer and a marine propeller mixing blade for 20 minutes at 800-1200 rpm. Formulations are listed in Table 24. Micrograms of the emulsions are shown in Figs. 1 to 3.

Table 24 -

| **Emulsification Properties; Formulations** | | | | |
|---|---|---|---|---|
| **Materials** | **Supplier** | **P - A** | **P - B** | **P - C** |
| **Part A** | | | | |
| Deionized Water -<br>Glycerin<br>Acrylates / Acrylamide / $C_{12-30}$ Polyoxyalkylene (meth)acrylate Crosspolymer (and) Mineral Oil (and) Polysorbate 85 | <br><br>Novemer™ EC-1 polymer (Noveon, Inc.) | 84.70<br>2.00<br>2.00* | 84.70<br>2.00 | 84.70<br>2.00 |
| **Part B** | | | | |
| Caprylic / Capric Triglyceride<br>C12-14 Alkyl Benzoate<br>Dimethicone<br>200 cP<br>Acrylates / Acrylamide / $C_{12-30}$ Polyoxyalkylene (meth)acrylate Crosspolymer (and) Mineral Oil (and) Polysorbate 85 | Liponate® GC (Lipo)<br>Finsolv® TN (Finetex)<br>Dow Corning® DC 200<br>Fluid (Dow Coming)<br>Novemer™ EC-1 polymer (Noveon, Inc.) | 5.00<br>5.00<br>1.00 | 5.00<br>5.00<br>1.00<br><br>2.00* | 5.00<br>5.00<br>1.00 |
| **Part C** | | | | |
| Acrylates / Acrylamide / $C_{12-30}$ Polyoxyalkylene (meth)acrylate Crosspolymer (and) Mineral Oil (and) Polysorbate 85 | *Novemer™ EC-1 polymer (Noveon, Inc.) | | | 2.00* |
| **Part D** | | | | |
| Pheonoxyethanol | Phenonip® (Clariant) | 0.30 | 0.30 | 0.30 |

* Novemer™ EC-1 polymer used as commercially supplied, in the formulation

Polymer Added to Water Phase

Referring to Fig. I, the polymer was added to the water phase prior to emulsification (Formulation P-A). The oil phase was added to the water/polymer phase with rapid agitation. The particle size of the oil droplets is uniform and approximately 10-15 µm. Microscopy completed using an Olympus BH-2 Microscope at 200X magnification. Olympus PM-10AK Camera.

Polymer Added to Oil Phase

In Fig. 2, the polymer was added to the oil phase prior to emulsification (Formulation P-B). The water phase was added to the oil/polymer phase with rapid agitation. The particle size of the oil droplets is uniform and approximately 10 - 15 µm. Microscopy completed using an Olympus BH-2 Microscope at 200X magnification. Olympus PM-10AK Camera.

Polymer Added to the Emulsion

In Fig. 3, the oil phase was added to the water phase with rapid agitation. The polymer was then added to the combined phases with rapid agitation (Formulation P-C). The particle size of the oil droplets is uniform and approximately 7-12.5 μm. Microscopy completed using an Olympus BH-2 Microscope at 200X magnification. Olympus PM-10AK Camera.

The foregoing experiments demonstrate that the salt tolerant inverse emulsion polymers of the present invention can be formulated under a wide variety of conditions forming stable emulsions. The order of addition of the inverse emulsion polymer of this invention in the formation of a multiphase emulsified system does not deleteriously affect the stability and emulsifying properties of the polymer. As is illustrated in Figs. 1 to 3, stable emulsions with uniform droplet sizes are obtained regardless of the phase in which the polymer is initially dispersed. These polymer attributes offer the formulator ease and versatility in the processing and manufacture of personal care compositions.

## Claims

**1.** A personal care emulsion composition for topical application to the skin comprising:

a) a continuous aqueous liquid phase;
b) a non-aqueous phase;
c) an electrolyte tolerant inverse emulsion polymer composition comprising:

i) a polymer polymerized from a monomer composition comprising (1) at least one pH sensitive monomer having ethylenic unsaturation and containing at least one carboxylic acid moiety, wherein at least 50 wt. % of said monomers containing said carboxylic acid moiety are neutralized; (2) a complex associative monomer represented by the structure:

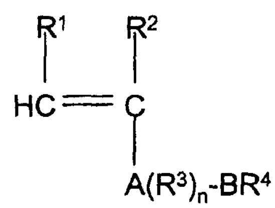

wherein A is selected from a divalent radical represented by $-C(O)O-$, $-CH_2C(O)O-$, -O-, $-CH_2O-$, NHC(O)O-, $-(CH_2)_mNHC(O)-$, -NHC(O)NH-, -C(O)NH-, $-C(O)NH(CH_2)_m-$,

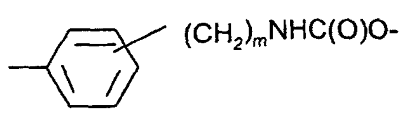

B is selected from a divalent radical represented by $-CH_2CH_2O-$, $-CH_2CH(CH_3)O-$, $-CH_2CH_2CH_2O-$, -C(O)-, $-(CH_2)_mNHC(O)-$, and $-(CH_2)_mNHC(O)NH-$, wherein m represents an integer from 0 to 18; $R^1$ is selected from H, $-CH_3$ and -COOH; $R^2$ is selected from H, $-CH_3$ and - COOH; $R^3$ is selected from $-CH_2CH_2O-$, $-CH_2CH(CH_3)O-$, $-CH_2CH_2CH_2O-$ and mixtures thereof wherein n is an integer from 1 to about 250; and $R^4$ is selected from substituted and unsubstituted linear and branched $C_8$-$C_{30}$ alkyl, substituted and unsubstituted $C_8$-$C_{40}$ alicyclic, $C_8$-$C_{30}$ alkylaryl wherein the aryl moiety contains 6 to 17 carbon atoms, $C_6$-$C_{17}$ arylalkyl wherein the alkyl moiety contains 8 to 30 carbon atoms, substituted and unsubstituted $C_5$-$C_{40}$ carbocylic groups; and (3) a crosslinking monomer; said complex associative monomer is present in the monomer composition in a weight ranging from 0.0001 wt. % to 3 wt. % based on the total weight of monomers present in the composition; and
ii) at least one surfactant having an HLB value less than 7; wherein said polymer maintains at least 50 %

of its original Brookfield Viscosity value when 0.1 wt. % of sodium chloride is added to 1 wt. % polymer solids in deionized water (w/w);

d) at least one adjuvant; and

e) a high HLB emulsifier having an HLB value of 7.5 or greater in an amount sufficient to allow said polymer in component to associate with said aqueous phase.

**2.** The composition of claim 1 wherein said monomer composition further contains a pH sensitive monomer selected from a monomer having ethylenic unsaturation and a sulfonic acid moiety, a monomer having ethylenic unsaturation and a neutralized sulfonic acid moiety, and mixtures thereof.

**3.** The composition of claim 1 wherein said monomer composition further contains a copolymerizable non-ionic monomer selected from $C_2$-$C_6$ hydroxy alkyl acrylates and methacrylates, glycerol monomethacrylate, tris(hydroxymethyl) ethane monoacrylate, pentaerythritol monomethacrylate, N-hydroxymethyl methacrylamide, hydroxyethyl methacrylamide, acrylamide, hydroxypropyl methacrylamide, vinyl caprolactam, N-vinyl pyrrolidone, $C_1$-$C_4$ alkoxy substituted methacrylates and methacrylamides, polyethylene glycol mono methacrylate and polypropylene glycol mono methacrylate, and mixtures thereof.

**4.** The composition of claim 1 wherein said complex associative monomer is selected from lauryl polyethoxylated methacrylate, palmityl polyethoxylated methacrylate, cetyl polyethoxylated methacrylate, cetylstearyl polyethoxylated methacrylate, stearyl polyethoxylated methacrylate, tristearylphenol polyethoxylated methacrylate, arachidyl polyethoxylated methacrylate, behenyl polyethoxylated methacrylate, cerotyl polyethoxylated methacrylate, montanyl polyethoxylated methacrylate, melissyl polyethoxylated methacrylate, lacceryl polyethoxylated methacrylate, and mixtures thereof.

**5.** The composition of claim 1 wherein said pH sensitive monomer is selected from acrylic acid, methacrylic acid, itaconic acid, citraconic acid, maleic acid, fumaric acid, crotonic acid, $C_1$-$C_4$ alkyl half esters of maleic, fumaric, itaconic acid, aconitic acid, and mixtures thereof.

**6.** The composition of claim 5 wherein 65 wt. % to 100 wt. % of the carboxylic acid groups on said pH sensitive monomer are neutralized.

**7.** The composition of claim 6 wherein said neutralized pH sensitive monomer is selected from the lithium, sodium, potassium, ammonium and amine salt of acrylic acid, methacrylic acid, itaconic acid, citraconic acid, maleic acid, fumaric acid, crotonic acid, $C_1$-$C_4$ alkyl half esters of maleic, fumaric, itaconic acid, aconitic acid, and mixtures thereof.

**8.** The composition of claim 2 wherein said monomer contains the lithium, sodium, potassium, ammonium and amine salt of said sulfonic acid.

**9.** The composition of claim 1 wherein said adjuvant is selected from humectants, emollients, biologically active materials, botanical extracts, conditioners, sunscreens, pharmaceutical actives, conditioning polymers, vitamins, cleansing surfactants, and mixtures thereof.

**10.** The composition of claim 1 wherein said high HLB surfactant is selected from high HLB $C_8$-$C_{22}$ alcohol ethoxylates, ethoxylated vegetable derived oils, ethoxylated $C_8$-$C_{22}$ fatty acids, $C_8$-$C_9$ alkyl phenol ethoxylates, esters of sorbitol, ethoxylated esters of sorbitol, and mixtures thereof

**11.** The composition of claim 1 wherein said crosslinking monomer contains at least two terminal $CH_2$=CH- groups.

**12.** The composition of claim 11 wherein said crosslinking monomer is selected from allyl pentaerythritol, methylene bis acrylamide, allyl sucrose, trimethylolpropane triacrylate, trimethylolpropane trimethacrylate, 1,6-hexanediol diacrylate, trimethylolpropane diallyl ether, pentaerythritol triacrylate, tetramethylene dimethacrylate, ethylene diacrylate, ethylene dimethacrylate, triethylene glycol dimethacrylate, and mixtures thereof.

**13.** The composition of claim 1 wherein said crosslinking monomer is present in an amount ranging from 0.0001 wt. % to 2 wt. % based on the total weight of monomers present in said monomer composition.

**14.** The composition of claim 3 wherein said non-ionic monomer is present in a range from about 1 to about 74.9998 based on the total weight of monomers present in said monomer composition.

**15.** The composition of claim 14 wherein said pH sensitive monomer mixture is present in the monomer composition in an amount ranging from about 25 wt. % to about 99.9998 wt. % based on the total weight of monomers present in said monomer composition.

**16.** The composition of claim 1, 2, 6 or 15 wherein said salt containing monomer is present in said pH sensitive monomer mixture in a range from 40 wt. % to 95 wt. % based on the total weight of said pH sensitive monomer mixture.

**17.** The composition of claim 1 or 9 wherein said composition further comprises a formulation aid selected from chelating agents, pH adjusters, opacifiers, preservatives, spreading agents, viscosity adjusters, rheology modifiers, film forming polymers, antioxidants, fragrances, colorants, pigments, glitter, pearlizing agents, and mixtures thereof.

**18.** The composition of claim 1 or 3 wherein said associative monomer is present in an amount ranging from 0.001 wt. % to 0.9 wt. % based on the total weight of monomers present in said monomer composition.

**19.** The composition of claim 1 wherein said polymer (i) is present in an amount ranging from 0.01 wt. % to 10 wt. % of the total weight of the personal care composition and is polymerized from a monomer composition comprising (1) from 25 wt. % to 99.9998 wt. % of said at least one pH sensitive monomer having ethylenic unsaturation and containing at least one carboxylic acid moiety, wherein at least 65 wt. % of said monomers containing said carboxylic acid moiety are neutralized; (2) from 0.0001 wt. % to 0.9 wt. % of said complex associative monomer; and (3) from 0.0001 wt. % to 2 wt. % of said crosslinking monomer; wherein said wt. % ranges are based on the total weight of monomers present in said monomer composition.

**20.** The composition of claim 1 or 10 wherein said high HLB surfactant is present in an amount ranging from about 0.2 wt. % to about 10 wt. % based on the total wt. of the monomers in said monomer composition.

**21.** The composition according to claim 19 further comprising:

at least one adjuvant selected from humectants, emollients, biologically active materials, botanical extracts, conditioners, sunscreens, pharmaceutical actives, conditioning polymers, vitamins, cleansing surfactants, and mixtures thereof.; and

a formulation aid selected from chelating agents, pH adjusters, opacifiers, preservatives, spreading agents, viscosity adjusters, rheology modifiers, film forming polymers, antioxidants, fragrances, colorants, pigments, glitter, pearlizing agents, and mixtures thereof.

**22.** The composition of claim 21 wherein said monomer composition comprises from 1 wt. % to 74.9998 wt. % of a non-ionic monomer selected from $C_2$-$C_6$ hydroxy alkyl acrylates and methacrylates, glycerol monomethacrylate, tris(hydroxymethyl) ethane monoacrylate, pentaerythritol monomethacrylate, N-hydroxymethyl methacrylamide, hydroxyethyl methacrylamide, acrylamide, hydroxypropyl methacrylamide, vinyl caprolactam, N-vinyl pyrrolidone, $C_1$-$C_4$ alkoxy substituted methacrylates and methacrylamides, polyethylene glycol mono methacrylate and polypropylene glycol mono methacrylate, and mixtures thereof.

**23.** The composition of claim 21 wherein said pH sensitive monomer is selected from acrylic acid and salts thereof, methacrylic acid and salts thereof, itaconic acid and salts thereof, citraconic acid and salts thereof, maleic acid and salts thereof, fumaric acid and salts thereof, crotonic acid and salts thereof, $C_1$-$C_4$ alkyl half esters of maleic, fumaric, itaconic acid, aconitic acid, and salts thereof; and mixtures thereof.

**24.** The composition of claim 21 wherein said wherein said monomer composition further contains a monomer selected from a monomer having ethylenic unsaturation and a sulfonic acid moiety, a monomer having ethylenic unsaturation and a salt of a sulfonic acid moiety, and mixtures thereof.

**25.** The composition of claim 21 wherein said pH sensitive monomer is selected from 2-acrylamido-2-methylpropane sulfonic acid, sodium p-styrene sulfonate, sulphoethyl methacrylate, and salts thereof; and mixtures thereof.

**26.** The composition of claim 24 wherein said pH sensitive monomer selected from sulfonic acid containing monomers, salts thereof, and mixtures thereof is present in said monomer composition in an amount ranging from 1 wt. % to

30 wt. % of the total pH sensitive monomer in said monomer composition.

**27.** The composition of claim 21 wherein said complex associative monomer is selected from lauryl polyethoxylated methacrylate, palmityl polyethoxylated methacrylate, cetyl polyethoxylated methacrylate, cetylstearyl polyethoxylated methacrylate, stearyl polyethoxylated methacrylate, tristearylphenol polyethoxylated methacrylate, arachidyl polyethoxylated methacrylate, behenyl polyethoxylated methacrylate, cerotyl polyethoxylated methacrylate, montanyl polyethoxylated methacrylate, melissyl polyethoxylated methacrylate, lacceryl polyethoxylated methacrylate, and mixtures thereof.

**28.** The composition of claim 27 wherein said complex associative monomer contains from about 10 to about 30 ethylene oxide units.

**29.** The composition of claim 21 wherein said crosslinking monomer is selected from allyl pentaerythritol, methylene bis acrylamide, allyl sucrose, trimethylolpropane triacrylate, trimethylolpropane trimethacrylate, 1,6-hexanediol diacrylate, trimethylolpropane diallyl ether, pentaerythritol triacrylate, tetramethylene dimethacrylate, ethylene diacrylate, ethylene dimethacrylate, triethylene glycol dimethacrylate, and mixtures thereof.

**30.** The composition of claim 21 wherein said non-aqueous phase is selected plant and vegetable oils, silicone oils, fluorinated hydrocarbon oils, hydrocarbon oils, mineral oils, polyisobutene, isohexadecane, caprylic/capric triglycerides, cetearyl octanoate, $C_{12}$-$C_{14}$ alkyl benzoate, and mixtures thereof.

**31.** The composition according to claim 1 comprising:

a) from 0.01 wt. % to 10 wt. % of said inverse emulsion polymer composition based on the total weight of the personal care composition;
b) at least one adjuvant selected from humectants, emollients, biologically active materials, botanical extracts, conditioners, sunscreens, pharmaceutical actives, conditioning polymers, vitamins, cleansing surfactants, and mixtures thereof;
c) a formulation aid selected from chelating agents, pH adjusters, opacifiers, preservatives, spreading agents, viscosity adjusters, rheology modifiers, film forming polymers, antioxidants, fragrances, colorants, pigments, glitter, pearlizing agents, and mixtures thereof; and
d) from 0.2 wt. % to 10 wt. % based on the total wt. of monomers in said monomer composition of a high HLB emulsifier having an HLB value of 7.5 or greater, wherein said inverse emulsion polymer composition comprises:

i) a polymer polymerized from a monomer composition comprising (1) from 25 wt. % to 99.9998 wt. % at least one pH sensitive monomer selected from acrylic acid and salts thereof, methacrylic acid and salts thereof, itaconic acid and salts thereof, citraconic acid and salts thereof, maleic acid and salts thereof, fumaric acid and salts thereof, crotonic acid and salts thereof, $C_1$-$C_4$ alkyl half esters of maleic, fumaric, itaconic acid, aconitic acid, and salts thereof; and mixtures thereof, where at least 65 wt. % of said monomers are neutralized; (2) from 0.001 wt. % to 0.75 wt. % of a complex associative monomer selected from lauryl polyethoxylated methacrylate, palmityl polyethoxylated methacrylate, cetyl polyethoxylated methacrylate, cetylstearyl polyethoxylated methacrylate, stearyl polyethoxylated methacrylate, tristearylphenol polyethoxylated methacrylate, arachidyl polyethoxylated methacrylate, behenyl polyethoxylated methacrylate, cerotyl polyethoxylated methacrylate, montanyl polyethoxylated methacrylate, melissyl polyethoxylated methacrylate, lacceryl polyethoxylated methacrylate, and mixtures thereof, wherein said polyethoxylated moiety contains from 10 to 30 ethylene oxide repeating units; (3) from 1 wt. % to 74.9998 wt. % of a non-ionic monomer selected from $C_2$-$C_6$ hydroxy alkyl acrylates and methacrylates, glycerol monomethacrylate, tris(hydroxymethyl) ethane monoacrylate, pentaerythritol monomethacrylate, N-hydroxymethyl methacrylamide, hydroxyethyl methacrylamide, acrylamide, hydroxypropyl methacrylamide, vinyl caprolactam, N-vinyl pyrrolidone, $C_1$-$C_4$ alkoxy substituted methacrylates and methacrylamides, polyethylene glycol mono methacrylate and polypropylene glycol mono methacrylate, and mixtures thereof; and (4) from 0.0001 wt. % to 2 wt. % based on the total weight of monomers present in the monomer composition of a crosslinking monomer; wherein said wt. % ranges are based on the total weight of monomers present in said monomer composition; and
ii) at least one surfactant having an HLB value less than 7; wherein said polymer maintains at least 25 % of its original Brookfield Viscosity value when 0.25 wt. % of sodium chloride is added to 1 wt. % polymer solids in deionized water (w/w).

**32.** The composition of claim 31 wherein said pH sensitive monomer is selected from acrylic acid and salts thereof, methacrylic acid and salts thereof, and mixtures thereof wherein from about 75 wt. % to about 95 wt. % of said monomers are neutralized.

**33.** The composition of claim 32 wherein said non-ionic monomer selected from N-hydroxymethyl methacrylamide, hydroxyethyl methacrylamide, acrylamide, hydroxypropyl methacrylamide, and mixtures thereof.

**34.** The composition of claim 31 wherein said crosslinking monomer is selected from alkyl pentaerythritol, methylene bis acrylamide, allyl sucrose, trimethylolpropane triacrylate, trimethylolpropane trimethacrylate, 1,6-hexanediol diacrylate, trimethylolpropane diallyl ether, pentaerythritol triacrylate, tetramethylene dimethacrylate, ethylene diacrylate, ethylene dimethacrylate, triethylene glycol dimethacrylate, and mixtures thereof.

**35.** The composition of claim 31 wherein said oil phase is a compound selected from mineral oil, vegetable oil, silicone oils, fluorinated hydrocarbon oils, hydrocarbon oils, and mixtures thereof.

**36.** The composition of claim 31 wherein said high HLB surfactant is selected from high HLB $C_8$-$C_{22}$ alcohol ethoxylates, ethoxylated vegetable derived oils, ethoxylated $C_8$-$C_{22}$ fatty acids, $C_8$-$C_9$ alkyl phenol ethoxylates, esters of sorbitol, ethoxylated esters of sorbitol, and mixtures thereof.

**Patentansprüche**

**1.** Körperpflege-Emulsionszusammensetzung zum topischen Auftragen auf die Haut, umfassend:

a) eine kontinuierliche wässrige, flüssige Phase;

b) eine nichtwässrige Phase;

c) eine elektrolyttolerante, inverse Emulsionspolymer-Zusammensetzung, umfassend:

i) ein Polymer, polymerisiert aus einer Monomerzusammensetzung, umfassend

(1) wenigstens ein pH-empfindliches Monomer mit einer ethylenischen Ungesättigtheit, das wenigstens einen Carbonsäurerest enthält, wobei wenigstens 50 Gew.-% der den Carbonsäurerest enthaltenden Monomere neutralisiert sind;

(2) ein komplexes, assoziatives Monomer, das durch die Struktur:

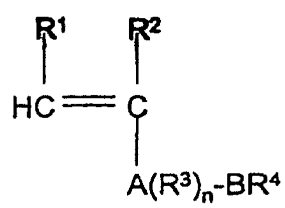

veranschaulicht ist, wobei A aus einem zweiwertigen Rest ausgewählt ist, der durch -C(O)O-, $-CH_2C$(O)O-, -O-, $-CH_2O-$, -NHC(O)O-, $-(CH_2)_mNHC(O)-$, -NHC(O)NH-, -C(O)NH-, $-C(O)NH(CH_2)_m-$,

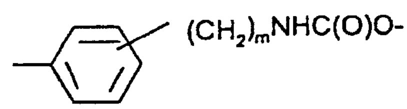

veranschaulicht ist,
B aus einem zweiwertigen Rest ausgewählt ist, der durch $-CH_2CH_2O-$, $-CH_2CH(CH_3)O-$, $-CH_2CH_2CH_2O-$, -C(O)-, $-(CH_2)_mNHC(O)-$ und $-(CH_2)_mNHC(O)NH-$veranschaulicht ist, wobei m eine ganze Zahl von 0 bis 18 darstellt, $R^1$ aus H, $-CH_3$ und -COOH ausgewählt ist, $R^2$ aus H, $-CH_3$ und -COOH ausgewählt ist, $R^3$ aus $-CH_2CH_2O-$, $-CH_2CH(CH_3)O$º, $-CH_2CH_2CH_2O-$und Mischungen davon ausgewählt ist, wobei n eine ganze Zahl von 1 bis etwa 250 ist, und $R^4$ aus einer substituierten und unsubstituierten, linearen und verzweigten $C_8$-$C_{30}$-Alkyl-, einer substituierten und unsubstituierten alicyclischen $C_8$-$C_{40}$-, einer $C_8$-$C_{30}$-Alkylaryl-, wobei der Arylrest 6 bis 17 Kohlenstoffatome enthält, einer $C_6$-$C_{17}$-Arylalkyl-, wobei der Alkylrest 8 bis 30 Kohlenstoffatome enthält, einer substituierten und unsubstituierten carbocyclischen $C_5$-$C_{40}$-Gruppe ausgewählt ist, und

(3) ein vernetzendes Monomer,

wobei das komplexe, assoziative Monomer in der Monomerzusammensetzung in einem von 0,0001 Gew.-% bis 3 Gew.-%, bezogen auf das Gesamtgewicht der in der Zusammensetzung vorhandenen Monomere, reichenden Gewicht vorhanden ist, und

ii) wenigstens ein Tensid mit einem HLB-Wert von weniger als 7, wobei das Polymer wenigstens 50 % seines ursprünglichen Wertes der Brookfield-Viskosität beibehält, wenn 0,1 Gew.-% Natriumchlorid zu 1 Gew.-% Polymerfeststoffen in deionisiertem Wasser (w/w) gegeben werden;

d) wenigstens ein Adjuvans und

e) ein Emulgator mit einem hohen HLB-Wert mit einem HLB-Wert von 7,5 oder mehr in einer Menge, die ausreichend ist, damit das Polymer in der Komponente mit der wässrigen Phase assoziieren kann.

**2.** Zusammensetzung nach Anspruch 1, wobei die Monomerzusammensetzung weiterhin ein pH-empfindliches Monomer enthält, das aus einem Monomer mit einer ethylenischen Ungesättigtheit und einem Sulfonsäurerest, einem Monomer mit einer ethylenischen Ungesättigtheit und einem neutralisierten Sulfonsäurerest und Mischungen davon ausgewählt ist.

**3.** Zusammensetzung nach Anspruch 1, wobei die Monomerzusammensetzung weiterhin ein copolymerisierbares, nichtionisches Monomer enthält, das aus $C_2$-$C_6$-Hydroxyalkylacrylaten und -methacrylaten, Glycerinmonomethacrylat, Tris(hydroxymethyl)ethanmonoacrylat, Pentaerythritmonomethacrylat, N-Hydroxymethylmethacrylamid, Hydroxyethylmethacrylamid, Acrylamid, Hydroxypropylmethacrylamid, Vinylcaprolactam, N-Vinylpyrrolidon, $C_1$-$C_4$-alkoxysubstituierten Methacrylaten und -methacrylamiden, Polyethylenglycolmonomethacrylat und Polypropylenglycolmonomethacrylat und Mischungen davon ausgewählt ist.

**4.** Zusammensetzung nach Anspruch 1, wobei das komplexe, assoziative Monomer aus laurylpolyethoxyliertem Methacrylat, palmitylpolyethoxyliertem Methacrylat, cetylpolyethoxyliertem Methacrylat, cetylstearylpolymethoxyliertem Methacrylat, stearylpolyethoxyliertem Methacrylat, tristearylphenolpolyethoxyliertem Methacrylat, arachidylpolyethoxyliertem Methacrylat, behenylpolyethoxyliertem Methacrylat, cerotylpolyethoxyliertem Methacrylat, montanylpolyethoxyliertem Methacrylat, melissylpolyethoxyliertem Methacrylat, laccerylpolyethoxyliertem Methacrylat und Mischungen davon ausgewählt ist.

**5.** Zusammensetzung nach Anspruch 1, wobei das pH-empfindliche Monomer aus Acrylsäure, Methacrylsäure, Itaconsäure, Citraconsäure, Maleinsäure, Fumarsäure, Crotonsäure, $C_1$-$C_4$-Alkylhalbestern von Malein-, Fumar-, Itaconsäure, Aconitsäure und Mischungen davon ausgewählt ist.

**6.** Zusammensetzung nach Anspruch 5, wobei 65 Gew.-% bis 100 Gew.-% der Carbonsäuregruppen im pH-empfindlichen Monomer neutralisiert sind.

**7.** Zusammensetzung nach Anspruch 6, wobei das neutralisierte, pH-empfindliche Monomer aus dem Lithium-, Natrium-, Kalium-, Ammonium- und Aminsalz von Acrylsäure, Methacrylsäure, Itaconsäure, Citraconsäure, Maleinsäure, Fumarsäure, Crotonsäure, $C_1$-$C_4$-Alkylhalbestern von Malein-, Fumar-, Itaconsäure, Aconitsäure und Mischungen davon ausgewählt ist.

**8.** Zusammensetzung nach Anspruch 2, wobei das Monomer das Lithium-, Natrium-, Kalium-, Ammonium- und Aminsalz der Sulfonsäure enthält.

**9.** Zusammensetzung nach Anspruch 1, wobei das Adjuvans aus Feuchthaltemitteln, Erweichungsmitteln, biologisch aktiven Materialien, botanischen Extrakten, Konditionierungsmitteln, Sonnenschutzfiltern, pharmazeutischen Wirkstoffen, konditionierenden Polymeren, Vitaminen, reinigenden Tensiden und Mischungen davon ausgewählt ist.

**10.** Zusammensetzung nach Anspruch 1, wobei das Tensid mit einem hohen HLB-Wert aus $C_8$-$C_{22}$-Alkoholethoxylaten, ethoxylierten, von pflanzlichen Substanzen stammenden Ölen, ethoxylierten $C_8$-$C_{22}$-Fettsäuren, $C_8$-$C_9$-Alkylphenolethoxylaten, Estern von Sorbit, ethoxylierten Estern von Sorbit mit einem hohen HLB-Wert und Mischungen davon ausgewählt ist.

**11.** Zusammensetzung nach Anspruch 1, wobei das vernetzende Monomer wenigstens zwei terminale $CH_2$=CH-Gruppen enthält.

**12.** Zusammensetzung nach Anspruch 11, wobei das vernetzende Monomer aus Allylpentaerythrit, Methylenbisacrylamid, Allylsaccharose, Trimethylolpropantriacrylat, Trimethylolpropantrimethacrylat, 1,6-Hexandioldiacrylat, Trimethylolpropandiallylether, Pentaerythrittriacrylat, Tetramethylendimethacrylat, Ethylendiacrylat, Ethylendimethacrylat, Triethylenglycoldimethacrylat und Mischungen davon ausgewählt ist.

**13.** Zusammensetzung nach Anspruch 1, wobei das vernetzende Monomer in einer von 0,0001 Gew.-% bis 2 Gew.-%, bezogen auf das Gesamtgewicht der in der Monomerzusarnmensetzung vorhandenen Monomere, vorhanden ist.

**14.** Zusammensetzung nach Anspruch 3, wobei das nichtionische Monomer in einem Bereich von etwa 1 bis 74,9998, bezogen auf das Gesamtgewicht der in der Monomerzusammensetzung vorhandenen Monomere, vorhanden ist.

**15.** Zusammensetzung nach Anspruch 14, wobei die pH-empfindliche Monomermischung in einer von etwa 25 Gew.-% bis etwa 99,9998 Gew.-% reichenden Menge, bezogen auf das Gesamtgewicht der in der Monomerzusammensetzung vorhandenen Monomere, in der Monomerzusamrnensetzung vorhanden ist.

**16.** Zusammensetzung nach Anspruch 1, 2, 6 oder 15, wobei das salzhaltige Monomer in der pH-empfindlichen Monomermischung in einem Bereich von 40 Gew.-% bis 95 Gew.-%, bezogen auf das Gesamtgewicht der pH-empfindlichen Monomermischung, vorhanden ist.

**17.** Zusammensetzung nach Anspruch 1 oder 9, wobei die Zusammensetzung weiterhin ein aus Komplexbildnern, pH-Stellmittein, Trübungsmitteln, Konservierungsmitteln, Verlaufmitteln, Viskositätsreglern, Rheologiereglern, filmbildenden Polymeren, Antioxidanzien, Duftstoffen, farbgebenden Mitteln, Pigmenten, Glitter, Perlmutterglanz verleihenden Mitteln und Mischungen davon ausgewähltes Formulierungshilfsmittel umfasst.

**18.** Zusammensetzung nach Anspruch 1 oder 3, wobei das assoziative Monomer in einer von 0,001 Gew.-% bis 0,9 Gew.-%, bezogen auf das Gesamtgewicht der in der Monomerzusammensetzung vorhandenen Monomere, vorhanden ist.

**19.** Zusammensetzung nach Anspruch 1, wobei das Polymer (i) in einer von 0,01 Gew.-% bis 10 Gew.-% des Gesamtgewichts der Körperpflege-Zusammensetzung reichenden Menge vorhanden ist und aus einer Monomerzusammensetzung polymerisiert wird, die (1) 25 Gew.-% bis 99,9998 Gew.-% wenigstens eines pH-empfindlichen Monomers mit einer ethylenischen Ungesättigtheit, das wenigstens einen Carbonsäurerest enthält, wobei wenigstens 65 Gew.-% der den Carbonsäurerest enthaltenden Monomere neutralisiert sind, (2) 0,0001 Gew.-% bis 0,9 Gew.-% des komplexen, assoziativen Monomers und (3) 0,0001 Gew.-% bis 2 Gew.-% des vernetzenden Monomers umfasst, wobei die Gew.-%-Bereiche auf das Gesamtgewicht der in der Monomerzusammensetzung vorhandenen Monomere bezogen sind.

**20.** Zusammensetzung nach Anspruch 1 oder 10, wobei das Tensid mit einem hohen HLB-Wert in einer von etwa 0,2 Gew.-% bis etwa 10 Gew.-% reichenden Menge, bezogen auf das Gesamtgewicht der Monomere in der Monomerzusammensetzung, vorhanden ist.

**21.** Zusammensetzung nach Anspruch 19, weiterhin umfassend:

wenigstens ein aus Feuchthaltemitteln, Erweichungsmitteln, biologisch aktiven Materialien, botanischen Extrakten, Konditionierungsmitteln, Sonnenschutzfiltern, pharmazeutischen Wirkstoffen, konditionierenden Polymeren, Vitaminen, reinigenden Tensiden und Mischungen davon ausgewähltes Adjuvans und

ein aus Komplexbildnern, pH-Stellmitteln, Trübungsmitteln, Konservierungsmitteln, Verlaufmitteln, Viskositätsreglern, Rheologiereglern, filmbildenden Polymeren, Antioxidanzien, Duftstoffen, farbgebenden Mitteln, Pigmenten, Glitter, Perlmutterglanz verleihenden Mitteln und Mischungen davon ausgewähltes Formulierungshilfsmittel.

**22.** Zusammensetzung nach Anspruch 21, wobei die Monomerzusammensetzung 1 Gew.-% bis 74,9998 Gew.-% eines nichtionischen Monomers umfasst, das aus $C_2$-$C_6$-Hydroxyalkylacrylaten und -methacrylaten, Glycerinmonomethacrylat, Tris(hydroxymethyl)ethanmonoacrylat, Pentaerythritmonomethacrylat, N-Hydroxymethylmethacrylamid, Hydroxyethylmethacrylamid, Acrylamid, Hydroxypropylmethacrylamid, Vinylcaprolactam, N-Vinylpyrrolidon, $C_1$-$C_4$-alkoxysubstutierten Methacrylaten und Methacrylamiden, Polyethylenglycolmonomethacrylat und Polypropylenglycolmonomethacrylat und Mischungen davon ausgewählt ist.

**23.** Zusammensetzung nach Anspruch 21, wobei das pH-empfindliche Monomer aus Acrylsäure und Salzen davon, Methacrylsäure und Salzen davon, Itaconsäure und Salzen davon, Citraconsäure und Salzen davon, Maleinsäure und Salzen davon, Fumarsäure und Salzen davon, Crotonsäure und Salzen davon, $C_1$-$C_4$-Alkylhalbestern von Malein-, Fumar-, Itaconsäure, Aconitsäure und Salzen davon und Mischungen davon ausgewählt ist.

**24.** Zusammensetzung nach Anspruch 21, wobei die Monomerzusammensetzung weiterhin ein Monomer enthält, das aus einem Monomer mit einer ethylenischen Ungesättigtheit und einem Sulfonsäurerest, einem Monomer mit einer ethylenischen Ungesättigtheit und einem Salz eines Sulfonsäuresalzes und Mischungen davon ausgewählt ist.

**25.** Zusammensetzung nach Anspruch 21, wobei das pH-empfindliche Monomer aus 2-Acrylamido-2-methylpropansulfonsäure, Natrium-p-styrolsulfonat, Sulfoethylmethacrylat und Salzen davon und Mischungen davon ausgewählt ist.

**26.** Zusammensetzung nach Anspruch 24, wobei das aus sulfonsäurehaltigen Monomeren, Salzen davon und Mischungen davon ausgewählte pH-empfindliche Monomer in der Monomerzusammensetzung in einer von 1 Gew.-% bis 30 Gew.-% reichenden Menge des gesamten pH-empfindlichen Monomers in der Monomerzusammensetzung vorhanden ist.

**27.** Zusammensetzung nach Anspruch 21, wobei das komplexe, assoziative Monomer aus laurylpolyethoxyliertem Methacrylat, palmitylpolyethoxyliertem Methacrylat, cetylpolyethoxyliertem Methacrylat, cetylstearylpolyethoxyliertem Methacrylat, stearylpolyethoxyliertem Methacrylat, tristearylphenolpolyethoxyliertem Methacrylat, arachidylpolyethoxyliertem Methacrylat, behenylpolyethoxyliertem Methacrylat, cerotylpolyethoxyliertem Methacrylat, montanylpolyethoxyliertem Methacrylat, melissylpolyethoxyliertem Methacrylat, laccerylpolyethoxyliertem Methacrylat und Mischungen davon ausgewählt ist.

**28.** Zusammensetzung nach Anspruch 27, wobei das komplexe, assoziative Monomer etwa 10 bis etwa 30 Ethylenoxid-Einheiten enthält.

**29.** Zusammensetzung nach Anspruch 21, wobei das vernetzende Monomer aus Allylpentaerythrit, Methylenbisacrylamid, Allylsaccharose, Trimethylolpropantriacrylat, Trimethylolpropantrimethacrylat, 1,6-Hexandioldiacrylat, Trimethylolpropandiallylether, Pentaerythrittriacrylat, Tetramethylendimethacrylat, Ethylendiacrylat, Ethylendimethacrylat, Triethylenglycoldimethacrylat und Mischungen davon ausgewählt ist.

**30.** Zusammensetzung nach Anspruch 21, wobei die nichtwässrige Phase aus pflanzlichen und tierischen Ölen, Siliconölen, fluorierten Kohlenwasserstoffölen, Kohlenwasserstoffölen, Mineralölen, Polyisobuten, Isohexadecan, Capryl-/Caprintriglyceriden, Cetearyloctanoat, $C_{12}$-$C_{14}$-Alkylbenzoat und Mischungen davon ausgewählt ist.

**31.** Zusammensetzung nach Anspruch 1, umfassend:

a) 0,01 Gew.-% bis 10 Gew.-% der inversen Emulsions-Polymerzusammensetzung, bezogen auf das Gesamtgewicht der Körperpflege-Zusammensetzung;

b) wenigstens ein aus Feuchthaltemitteln, Erweichungsmitteln, biologisch aktiven Materialien, botanischen Extrakten, Konditionierungsmitteln, Sonnenschutzfiltern, pharmazeutischen Wirkstoffen, konditionierenden Polymeren, Vitaminen, reinigenden Tensiden und Mischungen davon ausgewähltes Adjuvans und

c) ein aus Komplexbildnern, pH-Stellmitteln, Trübungsmitteln, Konservierungsmitteln, Verlaufmitteln, Viskositätsreglern, Rheologiereglern, filmbildenden Polymeren, Antioxidanzien, Duftstoffen, farbgebenden Mitteln, Pigmenten, Glitter, Perlmutterglanz verleihenden Mitteln und Mischungen davon ausgewähltes Formulierungshilfsmittel

d) 0,2 Gew.-% bis 10 Gew.-%, bezogen auf das Gesamtgewicht der Monomere in der Monomerzusammensetzung, eines Emulgators mit einem hohen HLB-Wert mit einem HLB-Wert von 7,5 oder mehr,

wobei die inverse Emulsions-Polymerzusammensetzung Folgendes umfasst:

i) ein Polymer, polymerisiert aus einer Monomerzusammensetzung, umfassend

(1) 25 Gew.-% bis 99,9998 Gew.-% wenigstens eines pH-empfindlichen Monomers, ausgewählt aus Acrylsäure und Salzen davon, Methacrylsäure und Salzen davon, Itaconsäure und Salzen davon, Citraconsäure und Salzen davon, Maleinsäure und Salzen davon, Fumarsäure und Salzen davon, Crotonsäure und Salzen davon, $C_1$-$C_4$-Alkylhalbestern von Malein-, Fumar-, Itaconsäure, Aconitsäure und Salzen davon und Mischungen davon, wobei wenigstens 65 Gew.-% der Monomere neutralisiert sind;

(2) 0,001 Gew.-% bis 0,75 Gew.-% eines komplexen, assoziativen Monomers, ausgewählt aus laurylpolyethoxyliertem Methacrylat, palmitylpolyethoxyliertem Methacrylat, cetylpolyethoxyliertem Methacrylat, cetylstearylpolyethoxyliertem Methacrylat, stearylpolyethoxyliertem Methacrylat, tristearylphenolpolyethoxyliertem Methacrylat, arachidylpolyethoxyliertem Methacrylat, behenylpolyethoxyliertem Methacrylat, cerotylpolyethoxyliertem Methacrylat, montanylpolyethoxyliertem Methacrylat, melissylpolyethoxyliertem Methacrylat, laccerylpolyethoxyliertem Methacrylat und Mischungen davon, wobei der polyethoxylierte Rest 10 bis 30 Ethylenoxid-Repetiereinheiten enthält;

(3) 1 Gew.-% bis 74,9998 Gew.-% eines nichtionischen Monomers, ausgewählt aus $C_2$-$C_6$-Hydroxyalkylacrylaten und -methacrylaten, Glycerinmonomethacrylat, Tris(hydroxymethyl)ethanmonoacrylat, Pentaerythritmonomethacrylat, N-Hydroxymethylmethacrylamid, Hydroxyethylmethacrylamid, Acrylamid, Hydroxypropylmethacrylamid, Vinylcaprolactam, N-Vinylpyrrolidon, $C_1$-$C_4$-alkoxysubstituierten Methacrylaten und -methacrylamiden, Polyethylenglycolmonomethacrylat und Polypropylenglycolmonomethacryat und Mischungen davon, und

(4) 0,0001 Gew.-% bis 2 Gew.-%, bezogen auf das Gesamtgewicht der in der Monomerzusammensetzung vorhandenen Monomere, eines vernetzenden Monomers, wobei die Gew.-%-Bereiche auf das Gesamtgewicht der in der Monomerzusammensetzung vorhanden Monomere bezogen sind, und

ii) wenigstens ein Tensid mit einem HLB-Wert von weniger als 7, wobei das Polymer wenigstens 25 % seines ursprünglichen Wertes der Brookfield-Viskosität behält, wenn 0,25 Gew.-% Natriumchlorid zu 1 Gew.-% Polymerfeststoffen in deionisiertem Wasser (w/w) gegeben werden.

**32.** Zusammensetzung nach Anspruch 31, wobei das pH-empfindliche Monomer aus Acrylsäure und Salzen davon, Methacrylsäure und Salzen davon und Mischungen davon ausgewählt ist, wobei etwa 75 Gew.-% bis etwa 95 Gew.-% der Monomere neutralisiert sind.

**33.** Zusammensetzung nach Anspruch 32, wobei das nichtionische Monomer aus N-Hydroxymethylmethacrylamid, Hydroxyethylmethacrylamid, Acrylamid, Hydroxypropylmethacrylamid und Mischungen davon ausgewählt ist.

**34.** Zusammensetzung nach Anspruch 31, wobei das vernetzende Monomer aus Alkylpentaerythrit, Methylenbisacrylamid, Allylsaccharose, Trimethylolpropantriacrylat, Trimethylolpropantrimethacrylat, 1,6-Hexandioldiacrylat, Trimethylolpropandiallylether, Pentaerythrittriacrylat, Tetramethylendimethacrylat, Ethylendiacrylat, Ethylendimethacrylat, Triethylenglycoldimethacrylat und Mischungen davon ausgewählt ist.

**35.** Zusammensetzung nach Anspruch 31, wobei die Ölphase eine Verbindung ist, die aus Mineralöl, pflanzlichem Öl,

Siliconölen, fluorierten Kohlenwasserstoffölen, Kohlenwasserstoffölen und Mischungen davon ausgewählt ist.

**36.** Zusammensetzung nach Anspruch 31, wobei das Tensid mit einem hohen HLB-Wert aus $C_8$-$C_{22}$-Alkoholethoxylaten, ethoxylierten, von pflanzlichen Substanzen stammenden Ölen, ethoxylierten $C_8$-$C_{22}$-Fettsäuren, $C_8$-$C_9$-Alkylphenolethoxylaten, Estern von Sorbit, ethoxylierten Estern von Sorbit mit einem hohen HLB-Wert und Mischungen davon ausgewählt ist.

## Revendications

**1.** Composition de soin personnel en émulsion pour une application topique sur la peau comprenant :

a) une phase aqueuse liquide continue ;
b) une phase non aqueuse ;
c) une composition de polymères en émulsion inverse tolérant un électrolyte comprenant :

i) un polymère polymérisé à partir d'une composition de monomères comprenant (1) au moins un monomère sensible au pH contenant une insaturation éthylénique et contenant au moins un groupement acide carboxylique, dans lequel au moins 50 % en poids desdits monomères contenant ledit groupement acide carboxylique sont neutralisés ; (2) un monomère d'association à un complexe représenté par la structure :

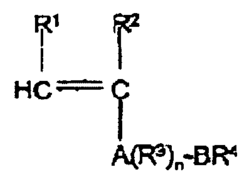

dans laquelle A est choisi parmi un radical divalent représenté par -C(O)O-, $-CH_2C(O)O-$, -O-, $-CH_2O-$, -NHC(O)O-, $-(CH_2)_mNHC(O)-$, -NHC(O)NH-, -C(O)NH-, $-C(O)NH(CH_2)_m-$,

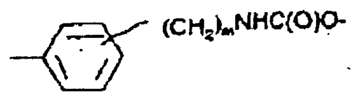

B est choisi parmi un radical divalent représenté par $-CH_2CH_2O-$, $-CH_2CH(CH_3)O-$, $-CH_2CH_2CH_2O-$, -C(O)-, $-(CH_2)_mNHC(O)-$ et $-(CH_2)_mNHC(O)NH-$ où m représente un nombre entier de 0 à 18 ; $R^1$ est choisi parmi H, $-CH_3$ et -COOH ; $R^2$ est choisi parmi H, $-CH_3$ et -COOH ; $R^3$ est choisi parmi $-CH_2CH_2O-$, $-CH_2CH(CH_3)O-$, $-CH_2CH_2CH_2O-$ et les mélanges de ceux-ci, où n est un nombre entier de 1 à environ 250 ; et $R^4$ est choisi parmi les groupes alkyle en $C_8$-$C_{30}$ substitué et non substitué, linéaire et ramifié, alicyclique en $C_8$-$C_{40}$ substitué et non substitué, alkylaryle en $C_8$-$C_{30}$ où le groupement aryle contient 6 à 17 atomes de carbone, arylalkyle en $C_6$-$C_{17}$ où le groupement alkyle contient 8 à 30 atomes de carbone, acide carboxylique en $C_5$-$C_{40}$ substitué et non substitué ; et (3) un monomère de réticulation; ledit monomère d'association à un complexe est présent dans la composition de monomères dans une proportion s'échelonnant de 0,0001 % en poids à 3 % en poids, par rapport au poids total des monomères présents dans la composition ; et
ii) au moins un tensioactif ayant une valeur HLB inférieure à 7 ; dans lequel ledit polymère conserve au moins 50 % de sa valeur de viscosité Brookfield d'origine lorsque 0,1 % en poids de chlorure de sodium est ajouté à 1 % en poids des matières solides du polymère dans de l'eau déminéralisée (en poids) ;

d) au moins un adjuvant ; et
e) un émulsifiant à HLB élevée ayant une valeur de HLB de 7,5 ou plus en une quantité suffisante pour permettre audit polymère dans le composant de s'associer à ladite phase aqueuse.

2. Composition selon la revendication 1, dans lequel ladite composition de monomères contient en outre un monomère sensible au pH choisi parmi un monomère comprenant une insaturation éthylénique et un groupement acide sulfonique, un monomère comprenant une insaturation éthylénique et un groupement acide sulfonique neutralisé, et les mélanges de ceux-ci.

3. Composition selon la revendication 1, dans lequel ladite composition de monomères contient en outre un monomère non ionique copolymérisable choisi parmi les méthacrylates et les acrylates d'hydroxyalkyle en $C_2$-$C_6$, le monométhacrylate de glycérol, le monoacrylate de tris(hydroxyméthyl)éthane, le monométhacrylate de pentaérythritol, le N-hydroxyméthylméthacrylamide, l'hydroxyéthylméthacrylamide, l'acrylamide, l'hydroxypropylméthacrylamide, le vinylcaprolactame, la N-vinylpyrrolidone, les méthacrylamides et les méthacrylates substitués par un groupe alcoxy en $C_1$-$C_4$, le monométhacrylate de polyéthylèneglycol et le monométhacrylate de polypropylèneglycol, et les mélanges de ceux-ci.

4. Composition selon la revendication 1, dans lequel ledit monomère d'association à un complexe est choisi parmi le méthacrylate de lauryle polyéthoxylé, le méthacrylate de palmityle polyéthoxylé, le méthacrylate de cétyle polyéthoxylé, le méthacrylate de cétylstéaryle polyéthoxylé, le méthacrylate de stéaryle polyéthoxylé, le méthacrylate de tristéarylphénol polyéthoxylé, le méthacrylate d'arachidyle polyéthoxylé, le méthacrylate de béhényle polyéthoxylé, le méthacrylate de cérotyle polyéthoxylé, le méthacrylate de montanyle polyéthoxylé, le méthacrylate de mélissyle polyéthoxylé, le méthacrylate de laccéryl polyéthoxylé, et les mélanges de ceux-ci.

5. Composition selon la revendication 1, dans lequel ledit monomère sensible au pH est choisi parmi l'acide acrylique, l'acide méthacrylique, l'acide itaconique, l'acide citraconique, l'acide maléique, l'acide fumarique, l'acide crotonique, les semi-esters alkyliques en $C_1$-$C_4$ des acides maléique, fumarique, itaconique et aconitique, et les mélanges de ceux-ci.

6. Composition selon la revendication 5, dans lequel 65 % en poids à 100 % en poids des groupes acide carboxylique sur ledit monomère sensible au pH sont neutralisés.

7. Composition selon la revendication 6, dans lequel ledit monomère sensible au pH neutralisé est choisi parmi le sel de lithium, de sodium, de potassium, d'ammonium et d'amine de l'acide acrylique, de l'acide méthacrylique, de l'acide itaconique, de l'acide citraconique, de l'acide maléique, de l'acide fumarique, de l'acide crotonique, des semi-esters alkyliques en $C_1$-$C_4$ des acides maléique, fumarique, itaconique et aconitique, et les mélanges de ceux-ci.

8. Composition selon la revendication 2, dans lequel ledit monomère contient le sel de lithium, de sodium, de potassium, d'ammonium et d'amine dudit acide sulfonique.

9. Composition selon la revendication 1, dans lequel ledit adjuvant est choisi parmi les humectants, les émollients, les matières actives au plan biologique, les extraits botaniques, les agents de conditionnement, les écrans solaires, les principes pharmaceutiques actifs, les polymères de conditionnement, les vitamines, les tensioactifs nettoyants, et les mélanges de ceux-ci.

10. Composition selon la revendication 1, dans lequel ledit tensioactif à HLB élevée est choisi parmi les éthoxylates d'alcool en $C_8$-$C_{22}$ à HLB élevée, les huiles dérivés de végétaux éthoxylés, les acides gras en $C_8$-$C_{22}$ éthoxylés, les éthoxylates d'alkylphénol en $C_8$-$C_9$, les esters de sorbitol, les esters éthoxylés de sorbitol, et les mélanges de ceux-ci.

11. Composition selon la revendication 1, dans lequel ledit monomère de réticulation contient au moins deux groupes $CH_2$=CH- terminaux.

12. Composition selon la revendication 11, dans lequel ledit monomère de réticulation est choisi parmi l'allylpentaérythritol, le méthylènebisacrylamide, l'allylsaccharose, le triacrylate de triméthylolpropane, le triméthacrylate de triméthylolpropane, le diacrylate de 1,6-hexanediol, l'éther diallylique de triméthylolpropane, le triacrylate de pentaérythritol, le diméthacrylate de tétraméthylène, le diacrylate d'éthylène, le diméthacrylate d'éthylène, le diméthacrylate de triéthylèneglycol, et les mélanges de ceux-ci.

13. Composition selon la revendication 1, dans lequel ledit monomère de réticulation est présent en une quantité s'échelonnant de 0,0001 % en poids à 2 % en poids, par rapport au poids total des monomères présents dans

ladite composition de monomères.

**14.** Composition selon la revendication 3, dans lequel ledit monomère non ionique est présent dans une plage d'environ 1 à environ 74,9998, par rapport au poids total des monomères présents dans ladite composition de monomères.

**15.** Composition selon la revendication 14, dans lequel ledit mélange de monomères sensibles au pH est présent dans la composition de monomères en une quantité s'échelonnant d'environ 25 % en poids à environ 99,9998 % en poids, par rapport au poids total des monomères présents dans ladite composition de monomères.

**16.** Composition selon la revendication 1, 2, 6 ou 15, dans lequel ledit monomère contenant un sel est présent dans ledit mélange de monomères sensibles au pH dans une plage de 40 % en poids à 95 % en poids, par rapport au poids total dudit mélange de monomères sensibles au pH.

**17.** Composition selon la revendication 1 ou 9, dans lequel ladite composition comprend en outre un auxiliaire de formulation choisi parmi les agents chélatants, les agents d'ajustement du pH, les opacifiants, les conservateurs, les agents d'étalement, les agents d'ajustement de la viscosité, les modificateurs de rhéologie, les polymères filmogènes, les antioxydants, les parfums, les colorants, les pigments, les agents réfléchissants, les agents coalescents, et les mélanges de ceux-ci.

**18.** Composition selon la revendication 1 ou 3, dans lequel ledit monomère d'association est présent en une quantité s'échelonnant de 0,001 % en poids à 0,9 % en poids, par rapport au poids total des monomères présents dans ladite composition de monomères.

**19.** Composition selon la revendication 1, dans lequel ledit polymère (i) est présent en une quantité s'échelonnant de 0,01 % en poids à 10 % en poids, du poids total de la composition de soin personnel et il est polymérisé à partir d'une composition de monomères comprenant (1) de 25 % en poids à 99,9998 % en poids dudit au moins un monomère sensible au pH ayant une insaturation éthylénique et contenant au moins un groupement acide carboxylique, dans lequel au moins 65 % en poids desdits monomères contenant ledit groupement acide carboxylique sont neutralisés ; (2) de 0,0001 % en poids à 0,9 % en poids dudit monomère d'association à un complexe ; et (3) de 0,0001 % en poids à 2 % en poids dudit monomère de réticulation ; dans lequel lesdites plages de pourcentage en poids sont rapportées au poids total des monomères présents dans ladite composition de monomères.

**20.** Composition selon la revendication 1 ou 10, dans lequel ledit tensioactif à HLB élevée est présent en une quantité s'échelonnant d'environ 0,2 % en poids à environ 10 % en poids, par rapport au poids total des monomères présents dans ladite composition de monomères.

**21.** Composition selon la revendication 19, comprenant en outre :

au moins un adjuvant choisi parmi les humectants, les émollients, les matières actives au plan biologique, les extraits botaniques, les agents de conditionnement, les écrans solaires, les principes pharmaceutiques actifs, les polymères de conditionnement, les vitamines, les tensioactifs nettoyants et les mélanges de ceux-ci ; et un auxiliaire de formulation choisi parmi les agents chélatants, les agents d'ajustement du pH, les opacifiants, les conservateurs, les agents d'étalement, les agents d'ajustement de la viscosité, les modificateurs de rhéologie, les polymères filmogènes, les antioxydants, les parfums, les colorants, les pigments, les agents réfléchissants, les agents coalescents, et les mélanges de ceux-ci.

**22.** Composition selon la revendication 21, dans lequel ladite composition de monomères comprend de 1 % en poids à 74,9998 % en poids d'un monomère non ionique choisi parmi les méthacrylates et les acrylates d'hydroxyalkyle en $C_2$-$C_6$, le monométhacrylate de glycérol, le monoacrylate de tris(hydroxyméthyl)éthane, le monométhacrylate de pentaérythritol, le N-hydroxyméthylméthacrylamide, l'hydroxyéthylméthacrylamide, l'acrylamide, l'hydroxypropylméthacrylamide, le vinylcaprolactame, la N-vinylpyrrolidone, les méthacrylamides et les méthacrylates substitués par un groupe alcoxy en $C_1$-$C_4$, le monométhacrylate de polyéthylèneglycol et le monométhacrylate de polypropylèneglycol, et les mélanges de ceux-ci.

**23.** Composition selon la revendication 21, dans lequel ledit monomère sensible au pH est choisi parmi l'acide acrylique et les sels de celui-ci, l'acide méthacrylique et les sels de celui-ci, l'acide itaconique et les sels de celui-ci, l'acide citraconique et les sels de celui-ci, l'acide maléique et les sels de celui-ci, l'acide fumarique et les sels de celui-ci, l'acide crotonique et les sels de celui-ci, les semi-esters alkyliques en $C_1$-$C_4$ des acides maléique, fumarique,

itaconique et aconitique et les sels de celui-ci, et les mélanges de ceux-ci.

**24.** Composition selon la revendication 21, dans lequel ladite composition de monomères contient en outre un monomère choisi parmi un monomère ayant une insaturation éthylénique et un groupement acide sulfonique, un monomère ayant une insaturation éthylénique et un sel d'un groupement acide sulfonique, et les mélanges de ceux-ci.

**25.** Composition selon la revendication 21, dans lequel ledit monomère sensible au pH est choisi parmi l'acide 2-acrylamido-2-méthylpropanesulfonique, le p-styrènesulfonate de sodium, le méthacrylate de sulfoéthyle et les sels de ceux-ci ; et les mélanges de ceux-ci.

**26.** Composition selon la revendication 24, dans lequel ledit monomère sensible au pH choisi parmi les monomères contenant de l'acide sulfonique, les sels de celui-ci, et les mélanges de celui-ci, est présent dans ladite composition de monomères en une quantité s'échelonnant de 1 % en poids à 30 % en poids du monomère sensible au pH total dans ladite composition de monomères.

**27.** Composition selon la revendication 21, dans lequel ledit monomère d'association à un complexe est choisi parmi le méthacrylate de lauryle polyéthoxylé, le méthacrylate de palmityle polyéthoxylé, le méthacrylate de cétyle polyéthoxylé, le méthacrylate de cétylstéaryle polyéthoxylé, le méthacrylate de stéaryle polyéthoxylé, le méthacrylate de tristéarylphénol polyéthoxylé, le méthacrylate d'arachidyle polyéthoxylé, le méthacrylate de béhényle polyéthoxylé, le méthacrylate de cérotyle polyéthoxylé, le méthacrylate de montanyle polyéthoxylé, le méthacrylate de mélissyle polyéthoxylé, le méthacrylate de laccéryl polyéthoxylé, et les mélanges de ceux-ci.

**28.** Composition selon la revendication 27, dans lequel ledit monomère d'association à un complexe contient d'environ 10 à environ 30 motifs oxyde d'éthylène.

**29.** Composition selon la revendication 21, dans lequel ledit monomère de réticulation est choisi parmi l'allylpentaérythritol, le méthylènebisacrylamide, l'allylsaccharose, le triacrylate de triméthylolpropane, le triméthacrylate de triméthylolpropane, le diacrylate de 1,6-hexanediol, l'éther diallylique de triméthylolpropane, le triacrylate de pentaérythritol, le diméthacrylate de tétraméthylène, le diacrylate d'éthylène, le diméthacrylate d'éthylène, le diméthacrylate de triéthylèneglycol, et les mélanges de ceux-ci.

**30.** Composition selon la revendication 21, dans lequel ladite phase non aqueuse est choisie parmi les huiles végétales et de plantes, les huiles de silicone, les huiles hydrocarbonées fluorées, les huiles hydrocarbonées, les huiles minérales, le polyisobutène, l'isohexadécane, les triglycérides capryliques/capriques, l'octanoate de cétéaryle, le benzoate d'alkyle en $C_{12}$-$C_{14}$, et les mélanges de ceux-ci.

**31.** Composition selon la revendication 1, comprenant :

a) de 0,01 % en poids à 10 % en poids de ladite composition de polymères en émulsion inverse, par rapport au poids totale de la composition de soin personnel ;
b) au moins un adjuvant choisi parmi les humectants, les émollients, les matières actives au plan biologique, les extraits botaniques, les agents de conditionnement, les écrans solaires, les principes pharmaceutiques actifs, les polymères de conditionnement, les vitamines, les tensioactifs nettoyants et les mélanges de ceux-ci ;
c) un auxiliaire de formulation choisi parmi les agents chélatants, les agents d'ajustement du pH, les opacifiants, les conservateurs, les agents d'étalement, les agents d'ajustement de la viscosité, les modificateurs de rhéologie, les polymères filmogènes, les antioxydants, les parfums, les colorants, les pigments, les agents réfléchissants, les agents coalescents, et les mélanges de ceux-ci ; et
d) de 0,2 % en poids à 10 % en poids, par rapport au poids des monomères dans ladite composition de monomères d'un émulsifiant à HLB élevée ayant une valeur HLB de 7,5 ou plus, dans laquelle ladite composition de polymères en émulsion inverse comprend ;

i) un polymère polymérisé à partir d'une composition de monomères comprenant (1) 25 % en poids à 99,9998 % en poids d'au moins un monomère sensible au pH choisi parmi l'acide acrylique et les sels de celui-ci, l'acide méthacrylique et les sels de celui-ci, l'acide itaconique et les sels de celui-ci, l'acide citraconique et les sels de celui-ci, l'acide maléique et les sels de celui-ci, l'acide fumarique et les sels de celui-ci, l'acide crotonique et les sels de celui-ci, les semi-esters alkyliques en $C_1$-$C_4$ des acides maléique, fumarique, itaconique et aconitique et les sels de celui-ci, et les mélanges de ceux-ci, où au moins 65 % en poids desdits monomères sont neutralisés ; (2) de 0,001 % en poids à 0,75 % en poids d'un monomère

d'association à un complexe choisi parmi le méthacrylate de lauryle polyéthoxylé, le méthacrylate de palmityle polyéthoxylé, le méthacrylate de cétyle polyéthoxylé, le méthacrylate de cétylstéaryle polyéthoxylé, le méthacrylate de stéaryle polyéthoxylé, le méthacrylate de tristéarylphénol polyéthoxylé, le méthacrylate d'arachidyle polyéthoxylé, le méthacrylate de béhényle polyéthoxylé, le méthacrylate de cérotyle polyéthoxylé, le méthacrylate de montanyle polyéthoxylé, le méthacrylate de mélissyle polyéthoxylé, le méthacrylate de laccéryl polyéthoxylé, et les mélanges de ceux-ci, où ledit groupement polyéthoxylé contient de 10 à 30 motifs récurrents oxyde d'éthylène ; (3) de 1 % en poids à 74,9998 % en poids d'un monomère non ionique choisi parmi les méthacrylates et les acrylates d'hydroxyalkyle en $C_2$-$C_6$, le monométhacrylate de glycérol, le monoacrylate de tris(hydroxyméthyl)éthane, le monométhacrylate de pentaérythritol, le N-hydroxyméthylméthacrylamide, l'hydroxyéthylméthacrylamide, l'acrylamide, l'hydroxypropylméthacrylamide, le vinylcaprolactame, la N-vinylpyrrolidone, les méthacrylamides et les méthacrylates substitués par un groupe alcoxy en $C_1$-$C_4$, le monométhacrylate de polyéthylèneglycol et le monométhacrylate de polypropylèneglycol, et les mélanges de ceux-ci ; et (4) de 0,0001 % en poids à 2 % en poids, par rapport au poids total des monomères présents dans la composition de monomères d'un monomère de réticulation, où lesdites plages de pourcentage en poids sont rapportées au poids total des monomères présents dans ladite composition de monomères ; et

ii) au moins un tensioactif ayant une valeur HLB inférieure à 7 ; dans lequel ledit polymère conserve au moins 25 % de sa valeur de viscosité Brookfield d'origine lorsque 0,25 % en poids de chlorure de sodium est ajouté à 1 % en poids des matières solides du polymère dans de l'eau déminéralisée (en poids).

**32.** Composition selon la revendication 31, dans lequel ledit monomère sensible au pH est choisi parmi l'acide acrylique et les sels de celui-ci, parmi l'acide méthacrylique et les sels de celui-ci, où d'environ 75 % en poids à environ 95 % en poids desdits monomères sont neutralisés.

**33.** Composition selon la revendication 32, dans lequel ledit monomère non ionique est choisi parmi le N-hydroxyméthylméthacrylamide, l'hydroxyéthylméthacrylamide, l'acrylamide, l'hydroxypropylméthacrylamide, et les mélanges de ceux-ci.

**34.** Composition selon la revendication 31, dans lequel ledit monomère de réticulation est choisi parmi l'allylpentaérythritol, le méthylènebisacrylamide, l'allylsaccharose, le triacrylate de triméthylolpropane, le triméthacrylate de triméthylolpropane, le diacrylate de 1,6-hexanediol, l'éther diallylique de triméthylolpropane, le triacrylate de pentaérythritol, le diméthacrylate de tétraméthylène, le diacrylate d'éthylène, le diméthacrylate d'éthylène, le diméthacrylate de triéthylèneglycol, et les mélanges de ceux-ci.

**35.** Composition selon la revendication 31, lequel ladite phase huileuse est un composé choisi parmi une huile minérale, une huile végétale, les huiles de silicone, les huiles hydrocarbonées fluorées, les huiles hydrocarbonées, et les mélanges de ceux-ci.

**36.** Composition selon la revendication 31, lequel ledit tensioactif à HLB élevée est choisi parmi les éthoxylates d'alcool en $C_8$-$C_{22}$ à HLB élevée, les huiles dérivés de végétaux éthoxylés, les acides gras en $C_8$-$C_{22}$ éthoxylés, les éthoxylates d'alkylphénol en $C_8$-$C_9$, les esters de sorbitol, les esters éthoxylés de sorbitol, et les mélanges de ceux-ci.

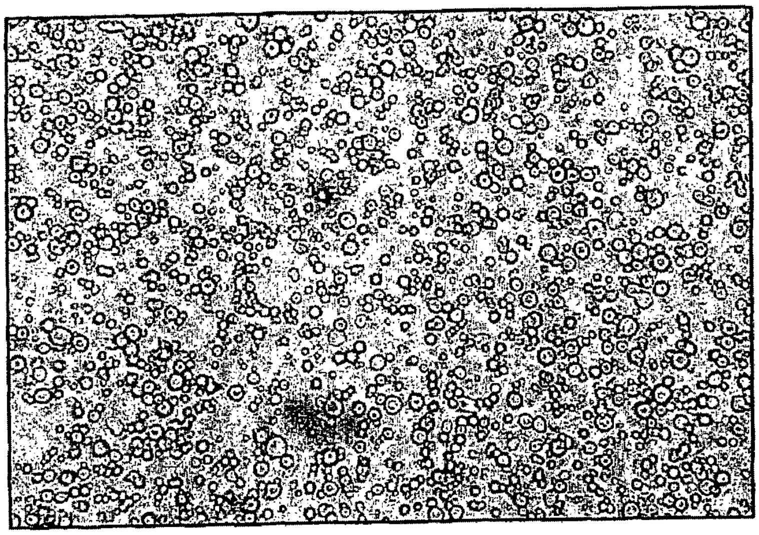

FIG-1

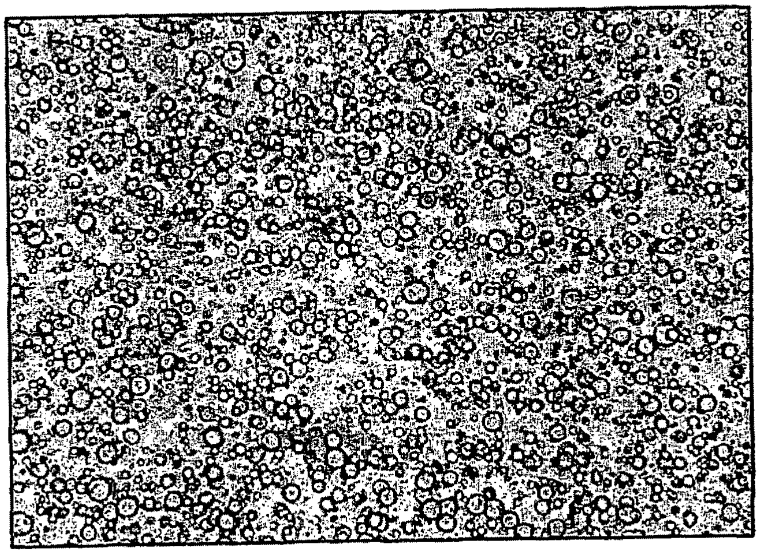

FIG-2

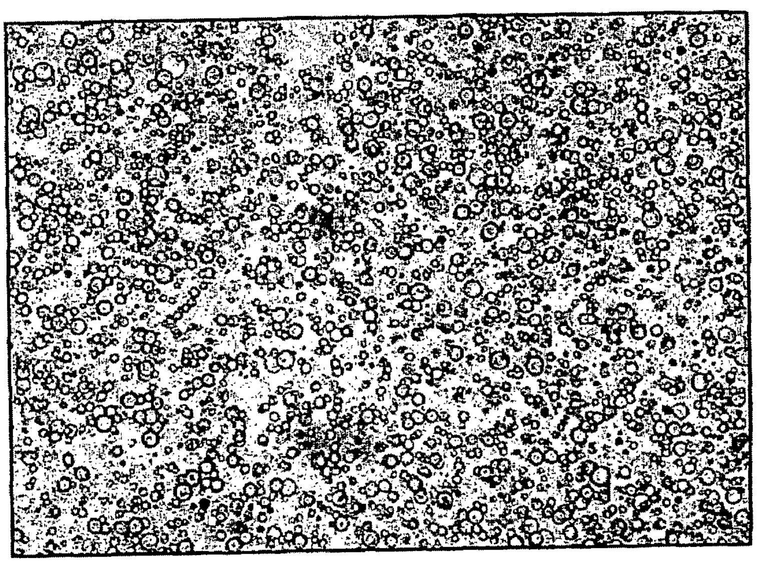

FIG-3